# EUROPEAN PATENT APPLICATION

(11) **EP 4 792 837 A1**
(43) Date of publication of application: **19.08.2026**
(21) Application number: 24876676.8
(22) Date of filing: 12.10.2024
(51) Int. Cl.: A61K 51/04, A61K 31/7088, C12N 15/113, A61K 103/00, A61K 47/54, A61P 35/00

(54) **CONJUGATE, DRUG CONJUGATE AND COMPOSITION, AND PREPARATION METHOD AND USE**

(30) Priority: 13.10.2023 CN 202311331221
(71) Applicant: Suzhou Ribo Life Science Co., Ltd., Suzhou, Jiangsu 215300 (CN)
(72) Inventor: LIANG, Zicai, Suzhou, Jiangsu 215300 (CN); GAO, Shan, Suzhou, Jiangsu 215300 (CN); GAN, Li Ming, Suzhou, Jiangsu 215300 (CN); CAO, Liqiang, Suzhou, Jiangsu 215300 (CN); WANG, Zhe, Suzhou, Jiangsu 215300 (CN)
(74) Representative: SSM Sandmair
(86) International application number: PCT/CN2024/124395
(87) International publication number: WO 2025/077871

(57) **Abstract**

Provided in the present disclosure is a conjugate comprising one or more delivery groups and one or more chelating agent groups. The chelating agent group is formed by removing one or more atoms or one or more functional groups from a chelating agent, the chelating agent being a chelating agent capable of forming a chelate with a radionuclide; and the delivery group is formed by removing one or more hydrogen atoms or one or more functional groups from an aptamer, the aptamer comprising a continuous sequence of nucleotides. Each of the delivery groups is independently connected to at least one of the chelating agent groups or other delivery groups via at least one linking group, and each of the chelating agent groups is independently connected to at least one of the delivery groups or at least one additional chelating agent group via at least one linking group. Also provided in the present disclosure is a drug conjugate comprising the conjugate described above and a nuclide. The drug conjugate provided in the present disclosure has a good tumor-targeting property and has a good application prospect.

## Description

### TECHNICAL FIELD

The present disclosure relates to a conjugate and a pharmaceutical composition comprising an aptamer-based delivery group and a functional group. The present disclosure also relates to the preparation methods and uses of these conjugates and pharmaceutical compositions.

### BACKGROUND ART

A tumor refers to a new growth formed by cell proliferation in local tissues under the action of various tumorigenic factors in the body. Such a new growth typically forms a local lump, from which it derives its name. Among them, tumor cells that metastasize and invade surrounding tissues are called malignant tumors. According to the classification of the source tissue cells of tumors, the tumors could be generally classified into malignant tumors derived from epithelial cells (cancer), malignant tumors derived from mesenchymal tissue cells (sarcoma), malignant tumors derived from blood stem cells (leukemia, etc.), and malignant tumors derived from glial cells (gliomas). Among them, glioma is the most common primary intracranial malignant tumor, accounting for approximately 40% to 50% of brain tumors, with an annual incidence of 3 to 8 cases per 100,000 persons worldwide. According to the WHO pathological classification standards, gliomas belong to neuroepithelial tumors, which include many pathological types, including but not limited to, pilocytic astrocytoma, diffuse astrocytoma, anaplastic astrocytoma, glioblastoma, oligodendroglioma, anaplastic oligodendroglioma, etc.

Currently, one of the important issues in the art of tumor treatment, especially glioma, is how to specifically deliver therapeutic agents to tumor tissues and cells and enable these therapeutic agents to produce corresponding therapeutic effects at the appropriate time and in the appropriate manner.

Aptamers, also known as nucleic acid aptamers, are oligonucleotide molecules that can bind to various molecules of interest, such as small molecule compounds, proteins, nucleic acids, and even cells, tissues and organs. The aptamers can provide the important property of "recognizing specific molecules", and thus, similar to antibodies, they are commonly used in biotechnology and therapy. The aptamers can be designed in test tubes and quickly synthesized by chemical methods. They also have the excellent properties of being easy to store and having low or no immunogenicity. Therefore, they have gradually gained attention from researchers in the art in recent years. However, the aptamers suitable for tumor-targeted delivery still need to be further developed and applied in the art.

### SUMMARY OF THE INVENTION

The inventors of the present disclosure have unexpectedly discovered a conjugate that could specifically target tumor cells, especially glioma cells. The conjugate shows high specificity to tumor cells, especially glioma cells, so that it could be effectively enriched in tumor cells, especially in glioma cells, and is suitable for the diagnosis and/or effective targeted therapy of tumors. Thus, the inventors have made the following invention:
In one aspect, the present disclosure provides a conjugate or a pharmaceutically acceptable salt thereof, wherein the conjugate comprises one or more delivery groups and one or more chelating agent-derived groups, wherein the chelating agent-derived group is formed by removing one or more atoms or one or more functional groups from a chelating agent, wherein the chelating agent being a chelating agent capable of forming a chelate with a radionuclide;
the delivery group is formed by removing one or more hydrogen atoms or one or more functional groups from an aptamer, wherein the aptamer comprises a segment of a contiguous nucleotide sequence, wherein the group linking two adjacent nucleotides is independently a phosphate ester group or a phosphate ester group with modification group(s), each nucleotide is one selected from modified or unmodified nucleotides, and the contiguous nucleotide sequence has the sequence as shown in Formula (1):

   5'- T₁-S₁-Nₐ-S₂-N_{b}-S₃-N_{c}-S₄-T₂ -3' Formula (1)

   wherein T₁ is a motif consisting of 1-3 nucleotides, T₂ is a motif consisting of 0-15 nucleotides, and T₂ does not comprise a motif that is completely reverse complementary to T₁;
S₁ and S₄ are each motifs consisting of 3-7 nucleotides, S₁ and S₄ are of the same length and are completely reverse complementary to each other;
Nₐ and N_{c} are each motifs consisting of 1-4 nucleotides, each nucleotide in Nₐ is not complementary to each nucleotide in N_{c}, and the total number of U in Nₐ and N_{c} accounts for more than 50% of the total number of all nucleotides in Nₐ and N_{c};
S₂ and S₃ are each motifs consisting of 1-4 nucleotides, S₂ and S₃ are of the same length and are completely reverse complementary to each other;
N_{b} is a motif consisting of 3-6 nucleotides, and the nucleotides at both ends of N_{b} do not form AU or GC complementarity;
each of the delivery groups is independently linked to at least one of the chelating agent-derived groups or other delivery groups via at least one linking group, and each of the chelating agent-derived groups is independently linked to at least one of the delivery groups or at least one additional chelating agent-derived group via at least one linking group.

In another aspect, the present disclosure also provides a pharmaceutical composition comprising the pharmaceutical conjugate of the present disclosure or a pharmaceutically acceptable salt thereof, and a pharmaceutically acceptable carrier.

In yet another aspect, the present disclosure also provides use of one or more of the pharmaceutical conjugate, the pharmaceutically acceptable salt and the pharmaceutical composition of the present disclosure in the manufacture of a medicament for diagnosing and/or treating tumors and tumor-related diseases or symptoms.

In yet another aspect, the present disclosure also provides a method for diagnosing and/or treating tumors and tumor-related diseases or symptoms, comprising administering an effective amount of one or more of the pharmaceutical conjugate, the pharmaceutically acceptable salt and the pharmaceutical composition of the present disclosure to a subject in need thereof.

In yet another aspect, the present disclosure further provides a kit comprising one or more of the pharmaceutical conjugate, the pharmaceutically acceptable salt and the pharmaceutical composition of the present disclosure.

### INCORPORATION BY REFERENCE

All publications, patents, and patent applications mentioned in this description are incorporated by reference into the present disclosure to the same extent as if each individual publication, patent, or patent application is specifically and individually incorporated by reference into the present disclosure.

### BENEFICIAL EFFECTS

The pharmaceutical conjugates and pharmaceutical compositions provided by the present disclosure have excellent ability to target tumors, especially glioma tissues and cells, and can significantly treat or alleviate tumors and tumor-related diseases and/or symptoms.

For example, in the glioma U118 subcutaneous tumor model mice, the pharmaceutical conjugates comprising a radionuclide as provided by the present disclosure exhibit enriched fluorescence signals in both subcutaneous U118 tumors of 300 mm³ and subcutaneous U118 tumors of 1,000 mm³, and with stronger fluorescence signals in the subcutaneous U118 tumors of larger area of 1,000 mm³, indicating that the pharmaceutical conjugates have higher enrichment and better targeting ability. For the mice inoculated with subcutaneous U118 tumors of 300 mm³, significant radiation signals were detected in the subcutaneous U118 tumors over a period of 24 hours; except that the metabolic organ kidney exhibited radiation signals, there was no significant enrichment in other organs. For another example, in the glioma U118 brain *in-situ* tumor model mice, the *in-situ* U118 tumors still showed significant radiation signals over a period of 48 hours after administration. For another example, after administration of the pharmaceutical conjugates comprising a radionuclide and a fluorophore as provided by the present disclosure, radiation signals could be detected in the subcutaneous U118 tumors over a period of 24 hours, indicating that the pharmaceutical conjugates provided by the present disclosure have excellent tumor-targeting ability, and can penetrate the blood brain barrier to specifically target tumors, and thus have good application prospects.

### BRIEF DESCRIPTION OF THE DRAWINGS

Figure 1 is a bar graph showing the *ex-vivo* quantitative analysis at 2h and 4h after injection of Conjugate RA1 into the model mice inoculated with subcutaneous U118 tumors of 300 mm³ in the Experimental Examples of the present disclosure.
Figure 2 is a bar graph showing the *ex-vivo* quantitative analysis at 2h and 4h after injection of Conjugate RA1 into the model mice inoculated with subcutaneous U118 tumors of 1,000 mm³ in the Experimental Examples of the present disclosure.
Figure 3 shows front-view SPECT-CT scan images of the *in-situ* tumor model mice in the experimental groups at 1h, 8h, 24h, and 48h post-administration in the Experimental Examples of the present disclosure.
Figure 4 shows top-view SPECT-CT scan images of the subcutaneous tumor model mice in the experimental groups at 0.5h, 1h, 4h, and 24h post-administration in the Experimental Examples of the present disclosure.
Figure 5 shows side-view SPECT-CT scan images of the subcutaneous tumor model mice in the control groups at 0.5h, 1h, 4h, and 24h post-administration in the Experimental Examples of the present disclosure.
Figure 6 shows top-view SPECT-CT scan images of the subcutaneous tumor model mice in the experimental groups at 0.5h, 1h, 4h, and 24h post-administration in the Experimental Examples of the present disclosure.

### DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure.

### Definition

In the present disclosure, unless otherwise specified, A, U, C, G and T respectively refer to adenosine nucleotide, uridine nucleotide, cytidine nucleotide, guanosine nucleotide, and thymidine nucleotide, and 2-methylcytosine nucleotide refers to a nucleotide obtained by substituting the 2' hydrogen on the cytosine base of the cytidine nucleotide with a methyl group. The structures of these nucleotides are well-known to those skilled in the art. As used herein, a "nucleic acid motif" or a "motif" refers to a nucleic acid sequence fragment in an oligonucleotide, consisting of one or more nucleotides. In some embodiments, a motif is a nucleic acid sequence fragment having a biological function.

As used herein, "alkyl" refers to straight-chain and branched chain saturated hydrocarbyl having the specified number of carbon atoms, typically from 1 to 20 carbon atoms, for example from 1 to 10 carbon atoms, such as from 1 to 8 or from 1 to 6 carbon atoms. For example, C₁-C₆ alkyl refers to both straight-chain and branched chain alkyl groups encompassing from 1 to 6 carbon atoms. When referring to an alkyl residue with a specific number of carbon atoms, it is intended to encompass all branched and straight-chain forms with that number of carbon atoms; thus, for example, "butyl" is meant to encompass n-butyl, sec-butyl, isobutyl and tert-butyl; "propyl" includes n-propyl and isopropyl. Alkylene is a subset of alkyl, referring to the same groups as alkyl, but having two attachment points.

As used herein, "alkenyl" refers to an unsaturated branched or straight-chain hydrocarbyl having one or more carbon-carbon double bonds obtained by respectively removing one hydrogen molecule from two adjacent carbon atoms of the parent alkyl. The group can be in either cis or trans configuration of the double bond(s). Typical alkenyl groups include, but are not limited to: ethenyl; propenyl, such as prop-1-en-1-yl, prop-1-en-2-yl, prop-2-en-1-yl (allyl), prop-2-en-2-yl; butenyl, such as, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms, while in other embodiments, from 2 to 10, from 2 to 8, or from 2 to 6 carbon atoms. Alkenylene is a subset of alkenyl, referring to the same groups as alkenyl, but having two attachment points.

As used herein, "alkynyl" refers to an unsaturated branched or straight-chain hydrocarbyl having one or more carbon-carbon triple bonds obtained by respectively removing two molecules of hydrogen from adjacent carbon atoms of the parent alkyl. Typical alkynyl groups include, but are not limited to, ethynyl; propynyl, such as, prop-1-yn-1-yl, prop-2-yn-1-yl; butynyl, such as, but-1-yn-1-yl, but-1-yn-3-yl, but-3-yn-1-yl; and the like. In certain embodiments, an alkynyl group has from 2 to 20 carbon atoms, and in other embodiments, from 2 to 10, 2 to 8, or 2 to 6 carbon atoms. Alkynylene is a subset of alkynyl, referring to the same groups as alkynyl, but having two attachment points.

As used herein, "heterocyclyl" refers to a stable 3- to 18-membered non-aromatic ring radical that comprises 2-12 carbon atoms and 1-6 heteroatoms selected from nitrogen, oxygen or sulfur. Unless otherwise stated in the description, the heteroaryl is a monocyclic, bicyclic, tricyclic, or tetracyclic ring system, which can include fused or bridged ring system(s). The heteroatom(s) in the heteroaryl can be oxidized heteroatom(s). One or more nitrogen atoms, if present, can be quaternized nitrogen atoms. The heterocyclyl is partially or fully saturated. The heteroaryl can be linked to the rest of the molecule through any ring atom. Examples of such heterocyclic groups include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Heterocyclylene is a subset of heterocyclyl, referring to the same groups as heterocyclyl , but having two attachment points.

As used herein, "aryl" refers to a radical derived from an aromatic monocyclic or multicyclic hydrocarbyl ring system by removing hydrogen atom(s) from a ring carbon atom. The aromatic monocyclic or multicyclic hydrocarbyl ring system comprises only hydrogen and carbon, including from 6 to 18 carbon atoms, wherein one or more rings in the ring system is fully unsaturated, i.e., it comprises a cyclic, delocalized (4n+2)π-electron system in accordance with the Hückel theory. Aryl groups include, but are not limited to, groups such as phenyl, fluorenyl, and naphthyl. Arylene is a subset of aryl, referring to the same groups as aryl, but having two attachment points.

"Heteroaryl" refers to a radical derived from a 3- to 18-membered aromatic ring free radical that comprises from 2 to 17 carbon atoms and 1 to 6 heteroatoms selected from nitrogen, oxygen or sulfur. As used herein, the heteroaryl can be a monocyclic, bicyclic, tricyclic, or tetracyclic ring system, wherein one or more rings in the ring system is fully unsaturated, i.e., it comprises a cyclic, delocalized (4n+2)π-electron system in accordance with the Hückel theory. Heteroaryl includes fused or bridged ring system(s). The heteroatom(s) in the heteroaryl can be oxidized heteroatom(s). One or more nitrogen atoms, if present, can be quaternized nitrogen atoms. The heteroaryl is linked to the rest of the molecule through any ring atom. Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10 hexahydrocycloocta[d]pyrimidinyl, 5,6,7,8,9,10-hexahydrocyclohepta[d]pyridazinyl, 5,6,7,8,9,10- hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl. Heteroarylene is a subset of heteroaryl, referring to the same groups as heteroaryl, but having two attachment points.

Unless otherwise specified, in the chemical formulae in the context of the present disclosure, an atom or group included in parentheses "()" represents that the atom or group is linked, outside the chain, to the atom adjacent to the left side of the parentheses in the chemical formula; that is, the atom or group is not linked in tandem inside the atom chain as shown in the chemical formula. For example, -C(O)-represents a carbonyl group; -CH₂O- represents a methylenedioxy group; -N(R₁)-represents an imino group substituted with R₁, and so on.

The term "subject", as used herein, refers to any animal, e.g., mammal or marsupial. The subject of the present disclosure includes, but is not limited to, human, non-human primate (e.g., rhesus or other kinds of macaque), mouse, pig, horse, donkey, cow, rabbit, sheep, rat, and any kind of poultry.

As used herein, the term "treatment" refers to a method for obtaining advantageous or desired result, including but not limited to, therapeutic benefit. "Therapeutic benefit" means eradication or amelioration of potential disorder to be treated. Also, therapeutic benefit is achieved by eradicating or ameliorating one or more of physiological symptoms associated with a potential disorder such that an amelioration is observed in a subject, although the subject may still be afflicted with the potential disorder.

### The conjugates provided by the present disclosure

In one aspect, the present disclosure provides a conjugate comprising one or more delivery groups and one or more chelating agent-derived groups. Each of the chelating agent-derived groups is independently formed by removing one or more atoms or one or more functional groups from a chelating agent, and the chelating agent is a chelating agent capable of forming a chelate with a radionuclide.

Each of the delivery groups is independently formed by removing one or more hydrogen atoms or one or more functional groups from an aptamer. Each of the delivery groups is independently linked to at least one of the chelating agent-derived groups or other delivery groups via at least one linking group, and each of the chelating agent-derived groups is independently linked to at least one of the delivery groups or at least one additional chelating agent-derived group via at least one linking group. The conjugate of the present disclosure can deliver functional groups to tumors.

In the conjugate provided by the present disclosure, the delivery group is formed by removing one or more hydrogen atoms or one or more functional groups from an aptamer, wherein the aptamer comprises a segment of a contiguous nucleotide sequence, wherein the group linking two adjacent nucleotides is independently a phosphate ester group or a phosphate ester group with modification group(s), each nucleotide is selected from one of modified or unmodified nucleotides, and the contiguous nucleotide sequence has the sequence as shown in Formula (1):

5'-T₁-S₁-Nₐ-S₂-N_{b}-S₃-N_{c}-S₄-T₂-3' Formula (1)

wherein T₁ is a motif consisting of 1-3 nucleotides. The inventors have found that the presence of T₁ can enable the conjugate provided by the present disclosure to exhibit highly efficient tumor-targeting effect. In some embodiments, T₁ consists of 2 nucleotides. In this case, the conjugate provided by the present disclosure has more excellent tumor-targeting capability. In some embodiments, T₁ consists of 2 nucleotides and comprises at least one C. In some embodiments, T₁ is CU, UC or AC in a 5'-3' direction.

T₂ is a motif consisting of 0-15 nucleotides. The inventors have found that T₂ with these numbers of nucleotides and with various nucleotide sequences does not significantly affect the tumor-targeting capability of the conjugate provided by the present disclosure. In some embodiments, T₂ is a motif consisting of 0-10 nucleotides.

In some embodiments, in a 5'-3' direction, T₂ consists of 1-9 nucleotides starting with U. In this case, the aptamer can have more excellent stability.

T₂ does not comprise the motif that is completely reverse complementary to T₁. In the context of the present disclosure, "reverse complementary" means that hydrogen bond linkages are formed between two nucleotide sequences or motifs according to the rules of nucleic acid base pairing, and each nucleotide of one nucleotide sequence or motif in a 5'-3' direction can sequentially form base pairs with each nucleotide of the other end nucleotide sequence or motif in a 3'-5' direction. In some embodiments, "reverse complementary" includes one or more of AU, GC, and UG complementarity.

S₁ and S₄ are each motifs consisting of 3-7 nucleotides, while S₁ and S₄ are of the same length and are completely reverse complementary to each other. The aptamer with the motifs S₁ and S₄ above has better stability and can target tumor tissues and cells over a long period of time. In some embodiments, S₁ and S₄ each consist of 3-5 nucleotides and are of the same length. In some embodiments, in the reverse complementarity formed between S₁ and S₄, GC complementarity accounts for more than 40% of the total number of complementarity. In this case, the conjugate provided by the present disclosure has further more excellent stability and tumor-targeting capability. In some embodiments, in a 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

Nₐ and N_{c} are each motifs consisting of 1-4 nucleotides. Each nucleotide in Nₐ is not complementary to each nucleotide in N_{c}, and the total number of U in Nₐ and N_{c} accounts for more than 50% of the total number of all nucleotides in Nₐ and N_{c}. The aptamer with the motifs Nₐ and N_{c} above shows excellent tumor tissue-targeting capability. In some embodiments, the sum of the number of nucleotides in Nₐ and N_{c} is an integer of 2-4. In some embodiments, the sum of the number of nucleotides in Nₐ and N_{c} is 3 or 4, and the sum of the number of U in Nₐ and N_{c} is 2 or 3. In some embodiments, in a 5'-3' direction, Nₐ and/or N_{c} are U, UU, UC or CU.

S₂ and S₃ are each motifs consisting of 1-4 nucleotides, while S₂ and S₃ are of the same length and are completely reverse complementary to each other. By comprising the motifs S₂ and S₃, the conjugate provided by the present disclosure shows good stability and excellent tumor-targeting capability. In some embodiments, S₂ and S₃ each consist of 2-3 nucleotides and are of the same length. In some embodiments, the reverse complementarity formed between S₂ and S₃ comprises at least one GC complementarity, and in this case, the reverse complementarity has better stability. In some embodiments, in a 5'-3' direction, S₂ is CA and S₃ is UG, or S₂ is AC and S₃ is GU, or S₂ is GCC and S₃ is GGU.

N_{b} is a motif consisting of 3-6 nucleotides, and the nucleotides at both ends of N_{b} do not form AU or GC complementarity. Without being limited by theory, the aptamer with the motif N_{b} above can maintain a specific configuration in terms of space, thereby enabling the conjugate provided by the present disclosure to stably and efficiently target tumor tissues and cells. In some embodiments, N_{b} consists of 4-5 nucleotides. In some embodiments, in a 5'-3' direction, N_{b} is GACG, GACGU, GACCG, UACU, GUUG or GAUCU.

The inventors of the present disclosure have unexpectedly found that the delivery group formed by the aptamer with the sequence as shown in Formula (1) above can effectively target tumors, especially glioma tissues, thereby allowing the conjugate provided by the present disclosure to specifically enter tumor cells and thus deliver therapeutic agents (such as chelating agent-derived groups) more effectively at cellular level.

In some embodiments, in the conjugate provided by the present disclosure, the contiguous nucleotide sequence has a length of 18-50 nucleotides, or 20-40 nucleotides, or 21-36 nucleotides, or 24-32 nucleotides. The delivery group formed by the aptamer having these lengths of the contiguous nucleotides and the conjugate comprising the delivery group as provided by the present disclosure can more easily target tumors and achieve a good balance between synthesis cost and targeting effect.

In some embodiments, in the conjugate provided by the present disclosure, the contiguous nucleotide sequences have the following sequences as shown in SEQ ID NO: 1, SEQ ID NO: 2, or SEQ ID NO: 3:
5'-CUGCUUCAGACGUGUUAGCUU-3' (SEQ ID NO: 1)
wherein in a 5'-3' direction, T₁ is CU, S₁is GCU, Nₐ is U, S₂ is CA, N_{b} is GACG, S₃ is UG, Nₒ is UU, S₄ is AGC, T₂ is UU;
5'-CUGAGUUCAGACGUGUUGCUCU-3' (SEQ ID NO: 2)
wherein in a 5'-3' direction, T₁ is CU, S₁ is GAGU, Nₐ is U, S₂ is CA, N_{b} is GACG, S₃ is UG, N_{c} is UU, S₄ is GCUC, T₂ is U;
5'-UCUAUGGCUGCCGAUCUGGUCUCCAUGUACGU-3' (SEQ ID NO: 3),
wherein in a 5'-3' direction, T₁ is CU, S₁ is GAGU, Nₐ is U, S₂ is CA, N_{b} is GACG, S₃ is UG, N_{c} is UU, S₄ is GCUC, T₂ is U.

In some embodiments, the contiguous nucleotide sequence has the nucleotide sequence as shown in SEQ ID NO: 4:
5'- N₆GGAGUUCAN₁N₂N₃N₄UGNsGCUCN₇ -3' (SEQ ID NO: 4),
wherein N₁, N₂ and N₃ independently of one another are one of A, U, C and G, N₄ is U, C or G or a motif consisting of two of U, C or G; N₅ is U, CU or UU; N₆ is CU, UC or AC; N₇ is U, UU or UUN₈, and N₈ is a motif consisting of 1-15 nucleotides.

In the nucleotide sequence above, in a 5'-3' direction, T₁ is the motif as shown by N₆, S₁ is the motif represented by GGAGU, Nₐ is U, S₂ is CA, N_{b} is the motif N₁N₂N₃N₄ consisting of N₁, N₂, N₃ and N₄, S₃ is UG, N_{c} is the motif represented by N₅, S₄ is the motif consisting of GCUC and the first nucleotide in N₇, and T₂ is the motif consisting of the remaining nucleotides in N₇.

The aptamer comprising the nucleotide sequence as shown in SEQ ID NO: 4 above can more effectively target tumors, especially gliomas, and can be enriched in tumor tissues.

Experimental verification shows that, in the nucleotide sequence as shown in SEQ ID NO: 4, the above selection of N₁, N₂, N₃ and N₄ does not significantly affect the tumor-targeting capability of the conjugate provided by the present disclosure. In some embodiments, the motif N₁N₂N₃N₄ consisting of N₁, N₂, N₃ and N₄ is one of GACG, GACGU, GACCG, UACU, GUUG, or GAUCU, and the aptamers comprising these motifs have higher tumor-specific targeting effects.

In some embodiments, in the nucleotide sequence as shown in SEQ ID NO: 4, N₅ is U or UU. In this case, the conjugates provided by the present disclosure all have excellent tumor-targeting effects.

In some embodiments, the aptamer has any one of the nucleotide sequences as shown in SEQ ID NOs: 5-11:
5'- CUGGAGUUCAGACGUGUUGCUCUU -3' (SEQ ID NO: 5)
5'- CUGGAGUUCAGACGUUGUGCUCUU -3' (SEQ ID NO: 6)
5'- CUGGAGUUCAGACCGUGUGCUCUU -3' (SEQ ID NO: 7)
5'- CUGGAGUUCAGACGUGUUGCUCU -3' (SEQ ID NO: 8)
5'- ACGGAGUUCAGACGUGUUGCUCUU -3' (SEQ ID NO: 9)
5'- CUGGAGUUCACUACUGUUGCUCUU -3' (SEQ ID NO: 10)
5'- CUGGAGUUCAGUUGUGUUGCUCUU -3' (SEQ ID NO: 11).

The conjugates having the nucleotide sequences above provided by the present disclosure show high tumor-targeting effects.

In some embodiments, the motif N₈ consists of 1-15 nucleotides. In some embodiments, N₈ consists of 1-8 nucleotides.

In some embodiments, the presence of the motif N₈ makes the conjugate provided by the present disclosure more stable against an *in vivo* exonuclease, thereby enabling it to exert the tumor-targeting effect *in vivo* for a long period of time. In some embodiments, N₈ can increase or maintain the tumor-targeting effect of the conjugate provided by the present disclosure. In some embodiments, the motif N₈ consists of 8 nucleotides, considering the balance among stability, targeting ability, and synthesis efficiency. In some embodiments, in a 5'-3' direction, the motif N₈ is CCGAUCUC. In some embodiments, the contiguous nucleotide sequence has any one of the nucleotide sequences as shown in SEQ ID NOs: 12-14:
5'- CUGGAGUUCAGACGUGUUGCUCUUCCGAUCUC -3' (SEQ ID NO: 12)
5'- CUGGAGUUCAGACGUUGUGCUCUUCCGAUCUC -3' (SEQ ID NO: 13)
5'- CUGGAGUUCAGACCGUGUGCUCUUCCGAUCUC -3' (SEQ ID NO: 14).

In the contiguous nucleotide sequence, the terminal groups at the 5' terminus of the ribose of the 5' terminal nucleotide and at the 3' terminus of the ribose of the 3' terminal nucleotide are independently hydroxyl or phosphate groups, and the selection of these terminal groups does not change the targeting capability of the conjugate provided by the present disclosure. In some embodiments, the terminal groups at the 5' terminus of the ribose of the 5' terminal nucleotide and at the 3' terminus of the ribose of the 3' terminal nucleotide are hydroxyl groups in the contiguous nucleotides.

In the conjugate provided by the present disclosure, each nucleotide can be a modified or unmodified nucleotide. In general, modifications of nucleotides can alter the stability and/or the tumor-targeting capability of the conjugate provided by the present disclosure. In some embodiments, at least one nucleotide in the conjugate provided by the present disclosure is a modified nucleotide. In some embodiments, at least one group linking two adjacent nucleotides in the conjugate provided by the present disclosure is a phosphate ester group with modification group(s).

The modification of nucleotide includes, but is not limited to, modification of sugar, modification of base, and/or substitution of a nucleotide with a nucleotide analog. In some embodiments, in the conjugate provided by the present disclosure, each of the modified nucleotides independently is one of 2'-halogen modified nucleotide, 2'-alkoxy modified nucleotide, 2'-alkyl modified nucleotide, 2'-substituted alkyl modified nucleotide, 2'-amino modified nucleotide, 2'-substituted amino modified nucleotide, 2'-deoxy nucleotide, a nucleotide with a modified base, and a nucleotide analog.

In the context of the present disclosure, a "fluoro modified nucleotide" refers to a nucleotide formed by substituting the 2'-hydroxy of the ribose group of the nucleotide with a fluoro group, which has the structure as shown in the following Formula (7). A "non-fluoro modified nucleotide" refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, or a nucleotide analogue.

These nucleotides formed by substituting the 2'-hydroxy of the ribose group with a non-fluoro group are well-known to those skilled in the art, and can be selected from one of 2'-alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide.

In some embodiments, the 2'-alkoxy modified nucleotide is the methoxy modified nucleotide (2'-OMe), as shown in Formula (8). In some embodiments, the 2'-amino modified nucleotide (2'-NH₂) is as shown in Formula (9). In some embodiments, the 2'-deoxy nucleotide (DNA) is as shown in Formula (10):

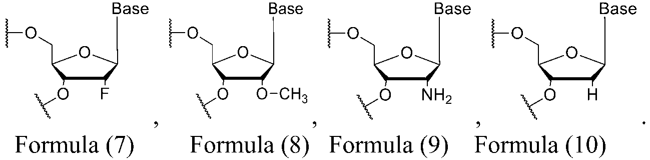

Those skilled in the art know various ways of modifying the bases of nucleotides. In some embodiments, base modification includes, but is not limited to, adding one or more methyl groups to a base. In some embodiments, thymine (T) is considered as one uracil (U) with a modified base. In some embodiments, 2-methylcytosine is considered as one cytosine (C) with a modified base.

A "nucleotide analogue" refers to a group that can replace a nucleotide in the nucleic acid, while structurally differs from an adenosine ribonucleotide, a guanosine ribonucleotide, a cytidine ribonucleotide, a uridine ribonucleotide or thymidine deoxyribonucleotide. In some embodiments, a nucleotide analogue can be an isonucleotide, a bridged nucleic acid (bridged nucleic acid, abbreviated as "BNA") nucleotide or an acyclic nucleotide.

A BNA refers to a nucleotide that is constrained or is not accessible. BNA can comprise a 5-, 6-membered or even a 7-membered ring bridged structure with a "fixed" C3'-endo sugar puckering. The bridge is typically incorporated at the 2'- and 4'-position of the ribose to afford a 2', 4'-BNA nucleotide. In some embodiments, BNA can be LNA, ENA and cET BNA, wherein LNA is as shown in Formula (12), ENA is as shown in Formula (13) and cET BNA is as shown in Formula (14).

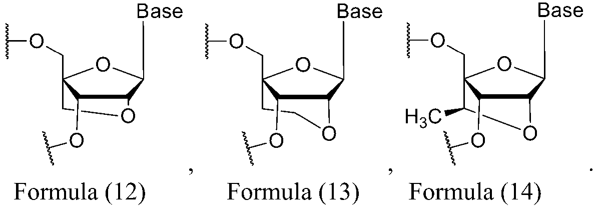

An acyclic nucleotide is a nucleotide in which the ribose ring is opened. In some embodiments, an acyclic nucleotide can be an unlocking nucleic acid (UNA), a glycerol nucleic acid (GNA) or a peptide nucleic acids (PNA), wherein UNA is as shown in Formula (15) and GNA is as shown in Formula (16):

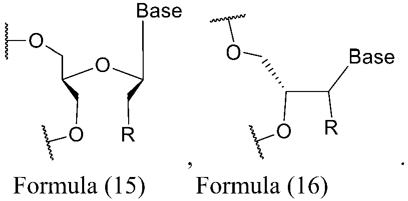

In the above Formula (15) and Formula (16), R is selected from H, OH, or alkoxy (O-alkyl).

A peptide nucleic acid is a nucleotide analog formed by replacing the glycoside-phosphate backbone with a polypeptide backbone. In some embodiments, the peptide nucleic acid can be, for example, a nucleotide analog formed by replacing the glycoside-phosphate unit with a 2-aminoethyl glycine bond.

An isonucleotide is a compound in which the position of the base on the ribose ring in the nucleotide is changed. In some embodiments, an isonucleotide can be the compound in which the base is transposed from position-1' to position-2' or -3' on the ribose ring, as shown in Formula (17) or (18), respectively.

In the above compounds of Formulae (17)-(18), "Base" represents a nucleic acid base, such as, A, U, G, C, or T; R is selected from H, OH, F, or a non-fluoro group above.

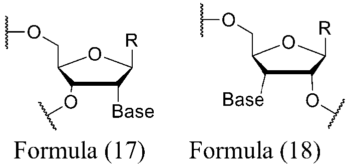

In some embodiments, a nucleotide analogue is selected from the group consisting of an isonucleotide, LNA, ENA, cET, UNA, and GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which 2'-hydroxy of the ribose group is substituted with a fluoro group" and a "nucleotide comprising 2'-fluororibosyl" have the same meaning, referring to the nucleotide formed by substituting the 2'-hydroxy of the ribose group with a fluoro group, having the structure as shown in Formula (7). A "methoxy modified nucleotide", a "2'-methoxy modified nucleotide", a "nucleotide in which 2'-hydroxy of a ribose group is substituted with a methoxy" and a "nucleotide comprising 2'-methoxyribosyl" have the same meaning, referring to a compound in which 2'-hydroxy of the ribose group in the nucleotide is substituted with a methoxy, and has the structure as shown in Formula (8).

In some embodiments, each cytidine nucleotide of the contiguous nucleotide sequence in the conjugate provided by the present disclosure is a fluoro modified cytidine nucleotide, and/or each uridine nucleotide of the contiguous nucleotide sequence in the conjugate provided by the present disclosure is a fluoro modified uridine nucleotide. In some embodiments, each nucleotide of the contiguous nucleotide sequence in the conjugate provided by the present disclosure is a 2'-methoxy modified nucleotide. In some embodiments, one or more uridine nucleotides in the conjugate provided by the present disclosure have modified bases. In some embodiments, a thymine base (T) is regarded as a uracil (U) base with a methyl modification.

The group linking two adjacent nucleotides can be a phosphate ester group or a modified phosphate ester group. The modification of a phosphate ester group can be, for example, substituting at least one non-bridging oxygen atom in a phosphate ester group with a sulfur atom to form a phosphorothioate group or a phosphorodithioate group. In some embodiments, at least one group linking two adjacent nucleotides in the conjugate provided by the present disclosure is a phosphorothioate group. In some embodiments, at least one of the three groups linking two adjacent nucleotides in the first four nucleotides at the 5' terminus in the contiguous nucleotide sequence is a phosphorothioate group. In some embodiments, at least two of the three groups linking two adjacent nucleotides in the first four nucleotides at the 5' terminus in the contiguous nucleotide sequence are phosphorothioate groups. In some embodiments, at least one of the groups linking two adjacent nucleotides in the first four nucleotides at the 3' terminus in the contiguous nucleotide sequence is a phosphorothioate group. In some embodiments, at least two of the three groups linking two adjacent nucleotides in the first four nucleotides at the 3' terminus in the contiguous nucleotide sequence are phosphorothioate groups. In some embodiments, each group linking two adjacent nucleotides in the contiguous nucleotide sequence is a phosphorothioate group.

The conjugate with the modification above provided by the present disclosure not only has low cost, but also can make the linking group less susceptible to cleavage by *in vivo* ribonuclease, thereby increasing the stability of the conjugate provided by the present disclosure and make it more resistant to nuclease hydrolysis. Meanwhile, the conjugate comprising the delivery group with the modification above as provided by the present disclosure has higher activity of targeting tumor tissues and/or cells.

In some embodiments, the contiguous nucleotide sequence has one of the nucleotide sequences as shown in SEQ ID NOs: 15-39:
5'- CfUfGGAGUfUfCfAGACfGUfGUfUfGCfUfCfUfUfCfCfGAUfCfUfCf -3' (SEQ ID NO: 15)
5'- CfUfGGAGUfUfCfAGACfGUfGUfUfGCfUfCfUfUf -3' (SEQ ID NO: 16)
5'- CfUfGGAGUfUfCfAGACfGUfUfGUfGCfUfCfUfUfCfCfGAUfCfUfCf -3' (SEQ ID NO: 17)
5'- CfUfGGAGUfUfCfAGACfGUfUfGUfGCfUfCfUfUf -3' (SEQ ID NO: 18)
5'- CfUfGGAGUfUfCfAGACfCfGUfGUfGCfUfCfUfUfCfCfGAUfCfUfCf -3' (SEQ ID NO: 19)
5'- CfUfGGAGUfUfCfAGACfCfGUfGUfGCfUfCfUfUf -3' (SEQ ID NO: 20)

5'- CmUmGmGmAmGmUmUmCmAmGmAmCmGmUmGmUmUmGmCmUmCm
UmUmCmCmGmAmUmCmUmCm -3' (SEQ ID NO: 21)

5'- CmUmGmGmAmGmUmUmCmAmGmAmCmGmUmGmUmGmCmUmCm
UmUm -3' (SEQ ID NO: 22)

5'- CmUmGmGmAmGmUmUmCmAmGmAmCmGmUmUmGmUmGmCmUmCm
UmUmCmCmGmAmUmCmUmCm -3' (SEQ ID NO: 23)

5'- CmUmGmGmAmGmUmUmCmAmGmAmCmGmUmUmGmUmGmCmUmCm
UmUm -3' (SEQ ID NO: 24)

5'- CmUmGmGmAmGmUmUmCmAmGmAmCmCmGmUmGmUmGmCmUmCm
UmUmCmCmGmAmUmCmUmCm -3' (SEQ ID NO: 25)

5'- CmUmGmGmAmGmUmUmCmAmGmAmCmCmGmUmGmUmGmCmUmCm
UmUm -3' (SEQ ID NO: 26)

5'- CmUmGmGmAmGmUmUmCmAmGmAmCmGmUmGmUmUmGmCmUmCm
Um -3' (SEQ ID NO: 27)

5'- CmUmGmAmGmUmUmCmAmGmAmCmGmUmGmUmUmGmCmUmCmUm -
3' (SEQ ID NO: 28)

5'-CmUmGmCmUmUmCmAmGmAmCmGmUmGmUmUmAmGmCmUmUm-3' (SEQ ID NO: 29)

5'- AmCmGmGmAmGmUmUmCmAmGmAmCmGmUmGmUmUmGmCmUmCm
UmUm -3' (SEQ ID NO: 30)

5'- CmUmGmGmAmGmUmUmCmAmCmUmAmCmUmGmUmUmGmCmUmCm
UmUm -3' (SEQ ID NO: 31)

5'- CmUmGmGmAmGmUmUmCmAmGmUmUmGmUmGmUmUmGmCmUmCm
UmUm -3' (SEQ ID NO: 32)

5'- UmCmUmAmUmGmGmCmUmGmCmCmGmAmUmCmUmGmGmUmCmUmC
mCmAmUmGmUmAmCmGmUm -3' (SEQ ID NO: 33)

5'- CmsUmsGmGmAmGmUmUmCmAmGmAmCmGmUmGmUmUmGmCmUmC
mUmUmCmCmGmAmUmCmsUmsCm -3' (SEQ ID NO: 34)

5'- CmsUmsGmGmAmGmUmUmCmAmGmAmCmGmUmGmUmUmGmCmUmC
msUmsUm -3' (SEQ ID NO: 35)

5'- CmsUmsGmGmAmGmUmUmCmAmGmAmCmGmUmUmGmUmGmCmUmC
mUmUmCmCmGmAmUmCmsUmsCm -3' (SEQ ID NO: 36)

5'- CmsUmsGmGmAmGmUmUmCmAmGmAmCmGmUmUmGmUmGmCmUmC
msUmsUm -3' (SEQ ID NO: 37)

5'- CmsUmsGmGmAmGmUmUmCmAmGmAmCmCmGmUmGmUmGmCmUmC
mUmUmCmCmGmAmUmCmsUmsCm -3' (SEQ ID NO: 38)

5'- CmsUmsGmGmAmGmUmUmCmAmGmAmCmCmGmUmGmUmGmCmUmC
msUmsUm -3' (SEQ ID NO: 39),

wherein C, G, U, and A represent the base composition of the nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage.

In some embodiments, the conjugate provided by the present disclosure has the structure as shown in Formula (101): 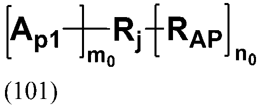
wherein each R_{AP} group is independently a group having the structure as shown in Formula (102): 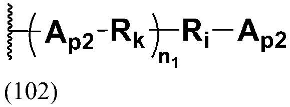
wherein each A_{P1} group and each A_{P2} group is identical or different, and independently and respectively represents one of the delivery groups or one of the chelating agent-derived groups, and the conjugate comprises at least one of the delivery groups and at least one of the chelating agent-derived groups; Rⱼ represents a linking group; each Rₖ or each Rᵢ is identical or different, and independently and respectively represents a covalent bond or a linking group, and Rᵢ and Rₖ are not covalent bonds at the same time; m₀ is an integer of 1 to 6; n₀ is an integer of 1 to 6; each n₁ independently of one another represents an integer of 0 to 4; and 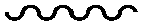 represents the site where the group is covalently linked.

In some embodiments, m₀ is an integer of 1 to 6, and A_{P1} is the chelating agent-derived group, that is, the conjugate represented by Formula (101) comprises 1 to 6 of the chelating agent-derived groups. Considering delivery efficiency and synthesis cost, in some embodiments, m₀ is an integer of 1 to 4, that is, the conjugate represented by Formula (101) comprises 1 to 4 of the chelating agent-derived groups. In some embodiments, m₀ is 1, that is, the conjugate represented by Formula (101) comprises one of the chelating agent-derived groups.

In some embodiments, n₀ is an integer of 1 to 6, that is, the conjugate represented by Formula (101) comprises 1 to 6 R_{AP} groups. Considering delivery efficiency and cost, in some embodiments, n₀ is an integer of 1 to 3, that is, the conjugate represented by Formula (101) comprises 1 to 3 R_{AP} groups. In some embodiments, n₀ is 1, that is, the conjugate represented by Formula (101) comprises one R_{AP} group.

In some embodiments, each n₁ independently of each other represents an integer of 0 to 4, and Rᵢ and Rₖ are not covalent bonds at the same time, and A_{P2} represents the delivery group, so that each R_{AP} group comprises 1 to 5 delivery groups. In some embodiments, each n₁ independently of each other represents an integer of 0 to 1, so that each R_{AP} group comprises 1-2 delivery groups. In some embodiments, n₀ is 1 and n₁ is 0; in this case, the conjugate represented by Formula (101) comprises one delivery group.

In the R_{AP} group, the function of Rₖ and Rᵢ is to covalently link the A_{P2} group to the Rⱼ group, and link to the A_{P1} group through the Rⱼ group. Therefore, as long as the above-mentioned linkage can be achieved, any Rₖ or Rᵢ that has no negative affect on the delivery group and the chelating agent-derived group can be used in the present invention. In some embodiments, each of the Rₖ or each of the Rᵢ is independently a straight-chain alkylene having a length of 1-70 carbon atoms, or one or more carbon atoms in the straight-chain alkylene are replaced by one or more groups selected from the group consisting of: C(O), NH, O, S, C(S), CH=N, S(O)₂, OP(O)₂, OP(O)(S), C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), - NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, - SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl). Among them, the oxygen or sulfur atom included in the parentheses of C(O), C(S), S(O)₂, OP(O)₂, and OP(O)(S) is linked to the adjacent atom via a double bond.

Considering synthesis cost and synthesis difficulty and the tumor-targeting effect of the conjugates, in some embodiments, each n₁ is 0, and each Rᵢ is independently a covalent bond, or one of the following linking group, or a linking combination of two or more of the following linking groups: C₁-C₂₀ alkylene, phosphate ester bond, phosphorothioate bond, amide bond, ester bond, ether bond, thioether bond, disulfide bond, 1,2,3-triazolylene group, a polyethylene glycol residue, pyrrolidinylene group, 2-oxopyrrolidinylene group, phenylene, cyclohexylene, 2-succinimidylene group, 2-thiosuccinimidylene group, an amino acid residue, and a nucleotide residue.

In some embodiments, the linking group Rⱼ comprises the linking groups that are well-known to those skilled in the art to be useful for antibody-conjugated drugs. The linking group Rⱼ can be cleavable or non-cleavable. In some embodiments, the linking group Rⱼ can be cleavable. In the context, "cleavable" means that after the conjugate of the present disclosure targets a tumor, the covalent bond of the linking group Rⱼ undergoes cleavage in the tumor environment and/or within tumor cells, releasing an individual therapeutic-agent group to produce a therapeutic effect. In some embodiments, the linking group Rⱼ comprises one or more of an active enzyme linking group, a sulfatase-cleavable linking group, a galactose-cleavable linking group, a lysosomal protease-sensitive linking group, a peptidyl linking group, a glucuronide linking group, an acid-sensitive cleavable linking group, or a glutathione-sensitive disulfide linking group. In some embodiments, the linking group Rⱼ comprises a peptidyl linking group. In some embodiments, the peptidyl linking group is selected from one or more of a valine-citrulline dipeptide linker (Val-Cit), an alanine-alanine dipeptide linker (Ala-Ala), a valine-alanine dipeptide linker (Val-Ala), a glycine-glycine-phenylalanine-glycine tetrapeptide linker (Gly-Gly-Phc-Gly). In some embodiments, the linking group Rⱼ is selected from one of N-succinimidyl 4-(2-pyridyldithio)butyrate (SPDB), N- succinimidyl-4-(2-thiopyridinylene) pentanoate (SPP), (S)-2-((S)-2-amino-3-methylbutanamido)-5-ureidopentanoic acid (Val-Cit-PAB-OH), N-succinimidyl-4-(N-maleimidomethyl)cyclohexane-1-carboxylate (SMCC) or 2-(phosphate-(CH₂)₆-S-)-maleimidohexanoyl-valine-citrulline-p-aminobenzylene. In some embodiments, the linking group Rⱼ comprises the linking groups listed in Mckertish CM, Kayser V. Advances and Limitations of Antibody Drug Conjugates for Cancer. Biomedicines. 2021 Jul 23; 9 (8): 872., which are incorporated herein by reference in their entirety.

In some embodiments, the linking group Rⱼ comprises one or more of a valine-citrulline dipeptide linker (Val-Cit), a polyethylene glycol residue, an iminohexylene, an N-succinimidyl group and a GAU trinucleotide linking group. In some embodiments, each Rᵢ is independently one of the following groups, or a linking combination of two or more of the following groups: a covalent bond, a disulfide bond, a dodecylene group, a valine-citrulline dipeptide linker (Val-Cit), a polyethylene glycol residue, an iminohexylene, an N-succinimidyl group or a GAU trinucleotide residue.

The function of the group Rⱼ is to link the R_{AP} group and the A_{P1} group; in some embodiments, A_{P1} is the chelating agent-derived group, and A_{P2} is the delivery group; and thereby the chelating agent-derived group A_{P1} is specifically delivered to tumor tissues and/or cells via the tumor-targeting effect of the delivery group A_{P2} in the R_{AP} group. Therefore, any Rⱼ group that is capable of achieving the above-mentioned linkage without affecting the tumor-targeting function of the delivery group and the effect of the chelating agent-derived group could achieve the purpose of the present invention and solve the technical problem to be solved by the present invention. In some embodiments, after the conjugate represented by Formula (1) reaches tumor tissues and/or enters tumor cells, the cleavage of Rⱼ takes place, releasing the pharmaceutically active molecule comprised in the individual chelating agent-derived group. In some embodiments, the Rⱼ is not cleaved *in vivo,* while the presence of the Rⱼ group and the R_{AP} group in the conjugate would not affect the therapeutic effect of the chelating agent-derived group.

In some embodiments, Rⱼ is a covalent bond, m₀ is 1; in this case, the conjugate represented by formula (101) comprises one A_{P1} group and one R_{AP} group, and each R_{AP} group is directly linked to the A_{P1} group. In some embodiments, each R_{AP} group is linked to the same atom of the A_{P1} group. In some embodiments, each R_{AP} group is linked to different atoms of the A_{P1} group.

In some embodiments, Rⱼ is a linking group, which comprises a backbone moiety, a side chain moiety, and a conjugation linking moiety.

The backbone moiety is respectively linked to the conjugation linking moiety and the side chain moiety. In some embodiments, the backbone moiety is a straight-chain alkylene group having a length of 1-70 carbon atoms, or one or more carbon atoms in the straight-chain alkylene group are replaced by one or more groups selected from the group consisting of: C(O), NH, O, S, C(S), CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), - NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, - SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl).

The side chain moiety is respectively linked to the backbone moiety and the R_{AP} group. In some embodiments, each side chain moiety is independently a covalent bond or a straight-chain alkylene group having a length of 1-70 carbon atoms, or one or more carbon atoms in the straight-chain alkylene group are replaced by one or more groups selected from the group consisting of: C(O), NH, O, S, C(S), CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and the straight-chain alkylene can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), - NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), - NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, - SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl).

The conjugation linking moiety is respectively linked to the backbone moiety and the Aₚ₁ group. In some embodiments, each conjugation linking moiety is independently a covalent bond, or one following linking structure, or a linking combination of two or more of the following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol residue, pyrrolidinylene group, 2-oxopyrrolidinylene group, phenylene, cyclohexylene, 2-succinimidylene group, 2-thiosuccinimidylene group, an amino acid residue, and a nucleotide residue.

In some embodiments, each of the conjugation linking moiety in the linking group Rⱼ is respectively linked to the backbone moiety and one of the Aₚ₁ groups; the number of side chain moieties is n₀ and each side chain moiety is respectively linked to the backbone moiety and one of the R_{AP} groups. Thus, each Aₚ₁ group and the R_{AP} group independently of one another are linked to a linking group Rⱼ. In some embodiments, all side chain moieties are linked to the same atom in the backbone moiety; alternatively, each side chain moiety is linked to different atoms in the backbone moiety.

In some embodiments, m₀ is 1, and the linking group Rⱼ comprises the structure as shown in Formula (301): 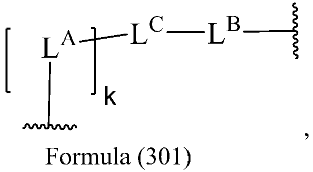 wherein k is an integer of 1 to 3; L^{C} is the backbone moiety, L^{A} is the side chain moiety, L^{B} is the conjugation linking moiety, and 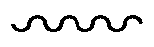 represents the site where the group is covalently linked.

The backbone moiety L^{C} is a covalent bond or a 2-4 valence, straight-chain or branched C₁-C₂₅ saturated hydrocarbyl, or one or more carbon atoms in the saturated hydrocarbyl are replaced by one or more groups selected from the group consisting of: C(O), NH, O, S, C(S), CH=N, S(O)₂, OP(O)₂, C₅-C₈ glycosidylene group, C₂-C₅ alkenylene group, C₂-C₅ alkynylene group, C₆-C₁₀ arylene group, C₃-C₈ heterocyclylene group, and C₅-C₁₀ heteroarylene group; and wherein the saturated hydrocarbyl can have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, - C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), - N(C₁-C₅ alkyl)C(O)(C₁-C₅ alkyl), -N(C₁-C₅ alkyl)C(O)(phenyl), -C(O)C₁-C₅ alkyl, - C(O)C₁-C₅ alkylphenyl, -OC(O)C₁-C₅ alkyl, -SO₂(C₁-C₅ alkyl), -SO₂(phenyl), - SO₂NH₂, -SO₂NH(C₁-C₅ alkyl), -SO₂NH(phenyl), -NHSO₂(C₁-C₅ alkyl), and - NHSO₂(phenyl). In some embodiments, L^{C} is a 2-4 valence, C₁-C₂₅ saturated hydrocarbyl, or one or more carbon atoms in the saturated hydrocarbyl are replaced by one or more groups selected from the group consisting of: C(O), NH, O, S, C(S), CH=N, S(O)₂, OP(O)₂, C₅-C₈ glycosidylene group, C₂-C₅ alkenylene group, C₂-C₅ alkynylene group, C₆-C₁₀ arylene group, C₃-C₈ heterocyclylene group, and C₅-C₁₀ heteroarylene group; and wherein the saturated hydrocarbyl can have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, nitro, and -CONH₂. In some embodiments, L^{C} is of 5-30 atoms in length, wherein the length of L^{C} refers to the number of chain-forming atoms in the longest chain of atoms formed by the atom directly linked to L^{A} to the atom directly linked to L^{B} in L^{C} To simplify the structure, in some embodiments, L^{C} is of 8-25 atoms in length.

The side chain moiety L^{A} is a covalent bond, or C₁-C₂₀ alkylene group, or one or more carbon atoms in the alkylene group are replaced by one or more groups selected from the group consisting of: C(O), NH, O, S, C(S), CH=N, S(O)₂, OP(O)₂, C₅-C₈ glycosidylene group, C₂-C₅ alkenylene group, C₂-C₅ alkynylene group, C₆-C₁₀ arylene group, C₃-C₈ heterocyclylene group, and C₅-C₁₀ heteroarylene group; and wherein the alkylene can have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-Cs alkyl-OH, -SC₁-C₅ alkyl, -SC₁-C₅ alkylphenyl, -C₁-C₅ alkyl-SH, -OH, -SH, -NH₂, - C₁-C₅ alkyl-NH₂, -N(C₁-C₅ alkyl)(C₁-C₅ alkyl), -NH(C₁-C₅ alkyl), -N(C₁-C₅ alkyl)(C₁-C₅ alkylphenyl), -NH(C₁-C₅ alkylphenyl), nitro, -C(O)O(C₁-C₅ alkyl), - CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O)(phenyl), -C(O)C₁-C₅ alkyl, -C(O)C₁-C₅ alkylphenyl, -OC(O)C₁-C₅ alkyl, - SO₂(C₁-C₅ alkyl), -SO₂(phenyl), -SO₂NH₂, -SO₂NH(C₁-C₅ alkyl), -SO₂NH(phenyl), - NHSO₂(C₁-C₅ alkyl), and -NHSO₂(phenyl).

The conjugation linking moiety L^{B} is one following linkage, or a linking combination of 2-5 following linkages: phosphate ester bond, phosphorothioate bond, amide bond, ester bond, ether bond, disulfide bond.

In some embodiments, k is an integer of 1 to 3; L^{C} comprises any one of the groups represented by formula (L1) - (L3), and is linked to L^{A} moiety through an ether bond in the group represented by formula (L1) - (L3): 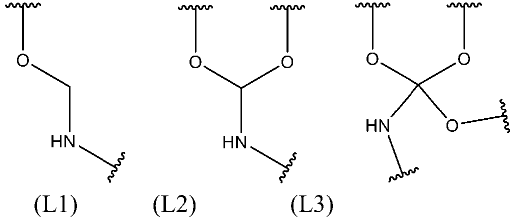 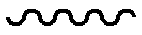 represents the site where a group is linked to the rest of the molecule.

In some embodiments, k = 1, L^{C} comprises a group represented by Formula (L1), and the oxygen atom in the group (L1) is directly linked to L^{A}. In some embodiments, k = 2, L^{C} comprises a group represented by Formula (L2), and each of two oxygen atoms in the group (L1) is directly linked to one L^{A}. In some embodiments, k = 4, L^{C} comprises a group represented by Formula (L3), and each of three oxygen atoms in the group (L3) is directly linked to one L^{A}

L^{B} is a phosphate ester bond or a disulfide bond.

Each L^{A} is a covalent bond, or each L^{A} is selected from the group consisting of the groups (L4)-(L23) and the linking combinations thereof: 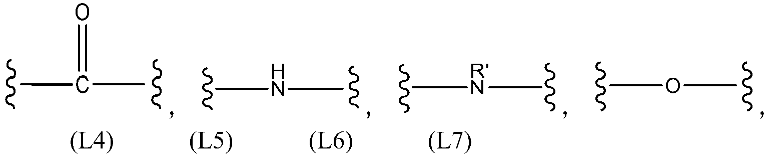 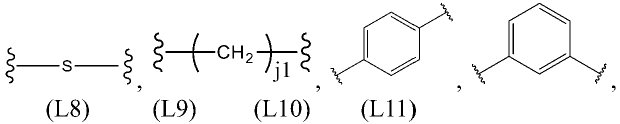 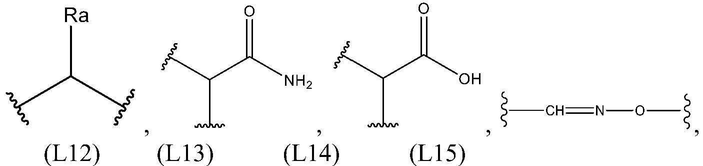 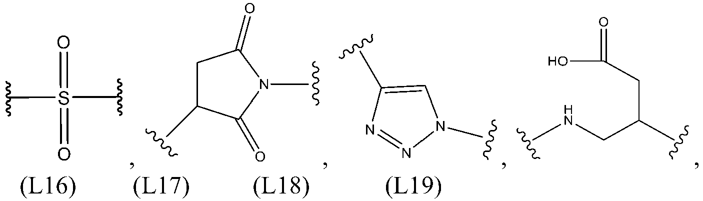 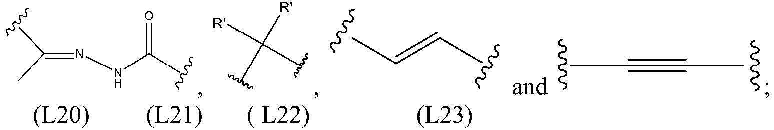
in the formula, each j1 is an integer of 1 to 10;
each R' is a C₁-C₁₀ alkyl;
each Ra is a hydrogen atom, C₁-C₁₀ alkyl, or selected from the group consisting of the groups (L24)-(L37):

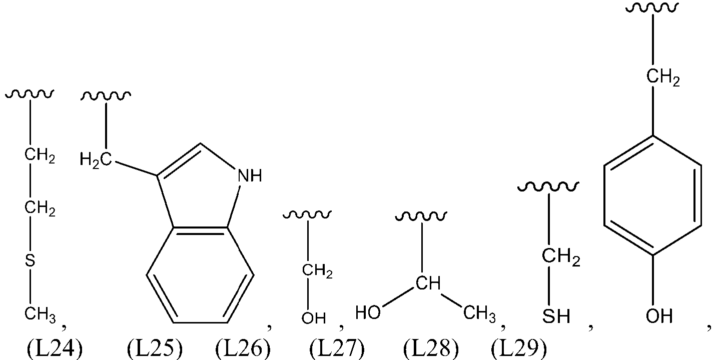

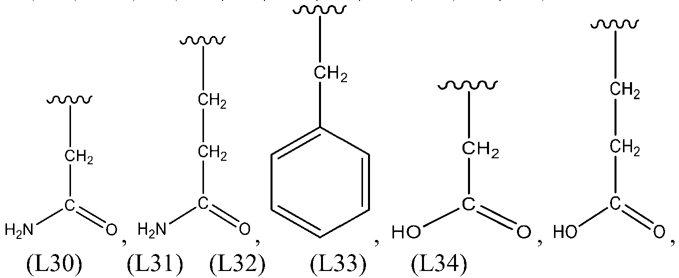

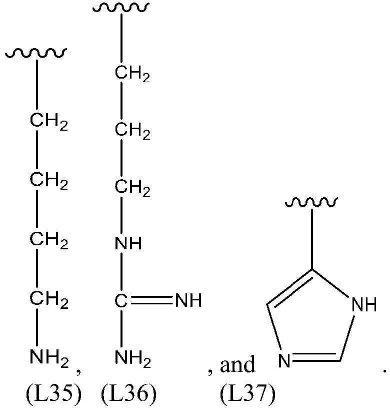

In some embodiments, L^{A} is of 3-35 atoms in length, wherein the length of the L^{A} refers to the number of chain-forming atoms in the longest chain of atoms formed by the atom directly linked to L^{C} to the atom directly linked to R_{AP} in L^{A}. In some embodiments, each L^{A} is a linking combination of at least two of the groups (L4) to (L9), (L13), (L14), and (L18). In some embodiments, each L^{A} is a linking combination of at least two of the groups (L4), (L5), (L7), (L9), (L13), (L14) and (L18).

In some embodiments, L^{A} has the amide bond-containing structure as shown in Formula (302); L^{B} has the N-acylpyrrolidine-containing structure as shown in Formula (303), comprising carbonyl groups and oxygen atoms; L^{C} is a linking group based on (hydroxymethyl)aminomethane, bis(hydroxymethyl)aminomethane, or tris(hydroxymethyl)aminomethane: 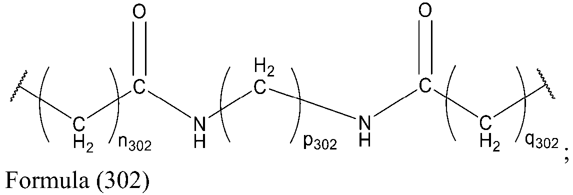 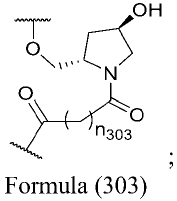 wherein n₃₀₂, q₃₀₂, and p₃₀₂ independently of one another are an integer of 2-6 and, optionally, n₃₀₂, q₃₀₂, and p₃₀₂ independently of one another are 2 or 3; n₃₀₃ is an integer of 4-16 and, optionally, n₃₀₃ is an integer of 8-12, and 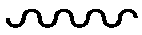 represents the site where the group is covalently linked.

In some embodiments, each of the side chain moieties L^{A} is linked to an R_{AP} group via a phosphate ester bond, an ether bond, or an ester bond, and is linked to said backbone moiety L^{C} by forming an ether bond with the oxygen atom of a hydroxyl group in the backbone moiety L^{C}; the conjugation linking moiety L^{B} is linked to the backbone moiety L^{C} by forming an amide bond between the nitrogen atom of the amino group in the backbone moiety L^{C} and the carbonyl group in Formula (303), and is linked to the Aₚ₁ group by forming a phosphate ester bond, an ether bond, or an ester bond between the oxygen atom in Formula (303) and the Aₚ₁ group. In some embodiments, the backbone moiety L^{C} is a linking group based on (hydroxymethyl)aminomethane, bis(hydroxymethyl)aminomethane, or tris(hydroxymethyl)aminomethane, and the backbone moiety L^{C} is linked to each of the side chain moieties L^{A} through an ether bond via the oxygen atom of the hydroxyl group, and is linked to the conjugation linking moiety L^{B} through an amide bond via the nitrogen atom of the amino group. Therefore, 1-3 side chains in the linking group Rⱼ are linked to the carbon atom of the same aminomethyl group, and are linked to delivery group-containing R_{AP} groups via the conjugation linking moiety L^{B}.

In some embodiments, the conjugate has the structure as shown in Formula (305):

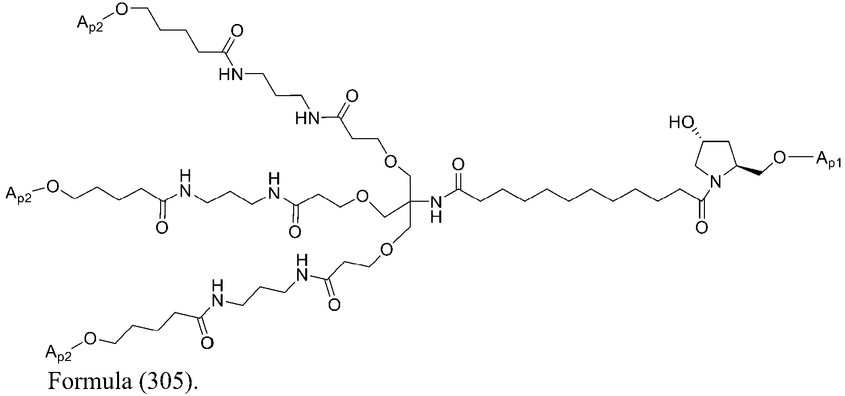

In some embodiments, the linking group Rⱼ comprises the structure as shown in Formula (306): 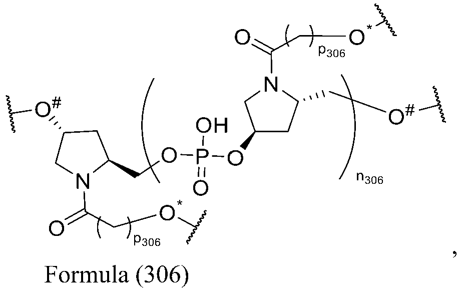 wherein n₃₀₆ is an integer of 0-3, each p₃₀₆ is independently an integer of 1-6, and 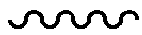 represents the site where the group is covalently linked; the linking combination formed by all pyrrolidinylene groups and any possible phosphodiester group constitutes the backbone moiety, the atomic chain between the carbonyl group linked to the nitrogen atom on a pyrrolidinylene group and an oxygen atom marked with * constitutes each side chain moiety, and the side chain moiety is linked to an R_{AP} group by forming an ether bond between the oxygen atom marked with * and the R_{AP} group; at least one of the oxygen atoms marked with # is a conjugation linking moiety and is linked to the Aₚ₁ group by forming an ether bond, an ester bond, or a phosphate ester bond with the Aₚ₁ group, and the other oxygen atom marked with # is linked to a hydrogen atom to form a hydroxyl group, or is linked to a C₁-C₃ alkyl group to form a C₁-C₃ alkoxy group. Therefore, 1-3 side chain moieties in the linking group Rⱼ are linked to different carbon atoms in the backbone moiety, and are linked to delivery-group-containing R_{AP} groups via an oxygen atom.

In some embodiments, the conjugate provided by the present disclosure has the structure as shown in Formula (307a), (307b), or (307c):

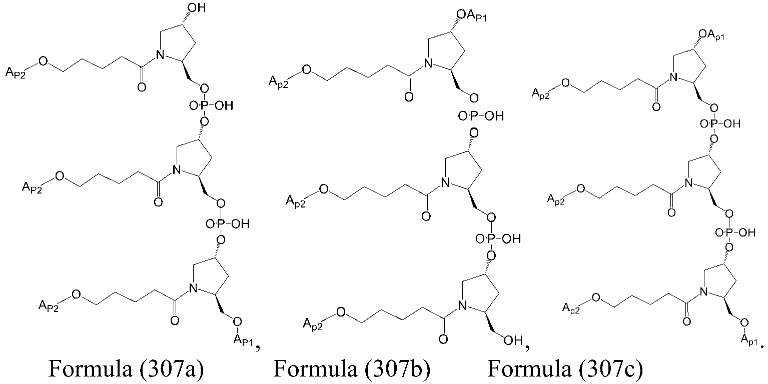

In some embodiments, the conjugate provided by the present disclosure has the structure as shown in Formula (308): 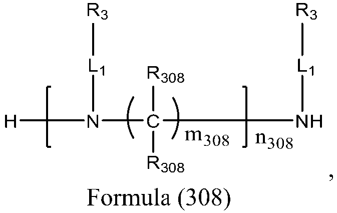 wherein n₃₀₈ can be an integer of 1-10; in some embodiments, in view of various factors such as synthesis convenience, structure/process costs, and tumor cell specificity, n₃₀₈ is an integer of 2-6. In some embodiments, n₃₀₈ is 3 or 4.

Each R₃ is independently the Aₚ₁ group, or the R_{AP} group. In some embodiments, at least one R₃ is the Aₚ₁ group, and at least one R₃ is the R_{AP} group. In some embodiments, one R₃ is the Aₚ₁ group, and the remaining R₃ groups are the R_{AP} groups.

In some embodiments, an Aₚ₂ group represents a delivery group, and when each m₃₀₈ is independently an integer of 2-10, it is considered that in the conjugate, the spatial position among multiple delivery groups A_{P2} can be more suitable for interaction with corresponding receptors on the surface of tumor cells. In order to make the compound as shown in Formula (308) have simpler structure, easier synthesis, and/or reduced costs, according to some embodiments of the present disclosure, each m₃₀₈ is independently an integer of 2-5; in some embodiments, each m₃₀₈ is equal.

In some embodiments, n₃₀₈ is an integer of 2-6, two to four of the R₃ groups are the diagnostic agent groups, and the remaining R₃ groups are the chelating agent-derived groups. In some embodiments, one R₃ is the chelating agent-derived group and the remaining R₃ groups are the diagnostic agent groups.

Those skilled in the art could understand that when each R₃₀₈ is independently selected from H, C₁-C₁₀ alkyl, C₁-C₁₀ haloalkyl, and C₁-C₁₀ alkoxy, they would not change the properties of the conjugate as shown in Formula (308) and could all realize the purpose of the present disclosure. In some embodiments, each R₃₀₈ is independently selected from H, methyl or ethyl. In some embodiments, each R₃₀₈ is H.

In some embodiments, an Aₚ₁ group represents a chelating agent-derived group, each L₁ linked to the Aₚ₁ group represents the conjugation linking moiety, and each L₁ linked to the R_{AP} group represents the side chain moiety. In some embodiments, one R₃ is the chelating agent-derived group Aₚ₁, and the remaining R₃ groups are the R_{AP} groups. In some embodiments, one or more L₁, as the side chain moieties, link the R_{AP} group to the N atom of the nitrogen-containing backbone; and the other one or more L₁, as the conjugation linking moieties, link the chelating agent-derived group Aₚ₁ to the N atom on the nitrogen-containing backbone. The nitrogen-containing backbones together form the backbone moiety of the linking group Rⱼ. In the context of the present disclosure, a "nitrogen-containing backbone" refers to the chain structure in the structure as shown in Formula (308), wherein the N atom is coadjacently linked to the carbon atom where R₃₀₈ is linked.

Considering delivery efficiency and synthesis costs, in some embodiments, each L₁ independently has a length of 3-25 atoms. In some embodiments, each L₁ independently has a length of 4-15 atoms. Those skilled in the art would understand that, though L₁ is defined as a linear alkylene for convenience, it may not be a linear group or may have different names, such as an amine or alkenylene produced by the above replacement and/or substitution. For the purpose of the present disclosure, the length of L₁ is the number of atoms in the chain linking the two attachment points. For this purpose, for the ring obtained by replacing a carbon atom in the straight-chain alkylene(e.g., a heterocyclylene or heteroarylene), the length of the portion in the chain corresponding to the ring is calculated according to the minimum number of atoms between attachment points on the ring.

In some embodiments, L₁ is selected from the group consisting of the groups as shown in Formulae L4-L23 above and any linking combination thereof. In some embodiments, each L₁ is independently selected from the group consisting of the linking combinations of at least two of the groups L4-L9, L13, L14, and L18. In some embodiments, each L₁ is independently a linking combination of at least two of the groups L4, L5, L7, L9, L13, L14, and L18.

In some embodiments, in the conjugate as shown in Formula (308), each L₁ has both an attachment site linked to the N atom on the nitrogen-containing backbone and an attachment linking site linked to Aₚ₁ or the R_{AP} group, and the site linked to the N atom on the nitrogen-containing backbone and the N atom form an amide bond. In some embodiments, at least one L₁ comprises a first attachment point and an optional second functional group, wherein the first attachment point and a group on the oligonucleotide or nucleotide form a phosphate ester bond or a phosphorothioate bond linkage, and the second functional group is a functional group formed after cleavage of the covalent linkage with the solid phase support. In some embodiments, the first attachment point is selected from an oxygen atom or a sulfur atom. In some embodiments, the second functional group is a hydroxyl group or an amino group. In some embodiments, one or more L₁ groups are selected from B5, B6, B5', or B6': 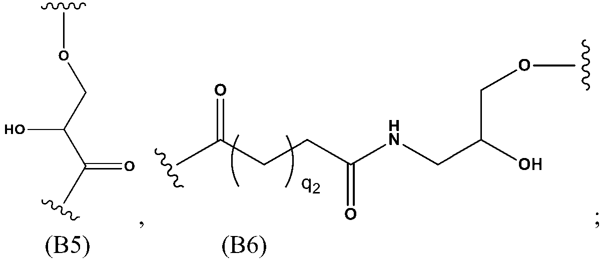 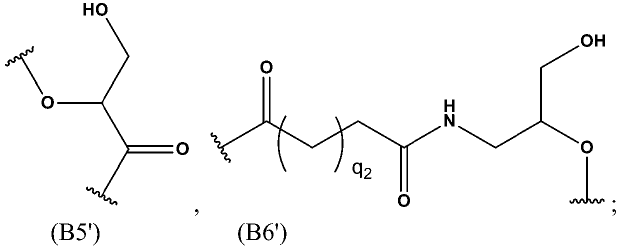 wherein 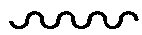 represents the site where the group is covalently linked, and q₂ is an integer of 1-10. In some embodiments, q₂ is an integer of 1-5.

In some embodiments, each R_{AP} group comprises one or more delivery groups. In some embodiments, the compound as shown in Formula (308) comprises multiple chelating agent-derived groups.

In some embodiments, the compound as shown in Formula (308) has the structure as shown in Formula (403), (404), (405), (406), (407), (408), (409), (410), (411), (412), (413), (414), (415), (416), (417), (418), (419), (420), (421), (422), (423), (424), (425), (426), or (427): 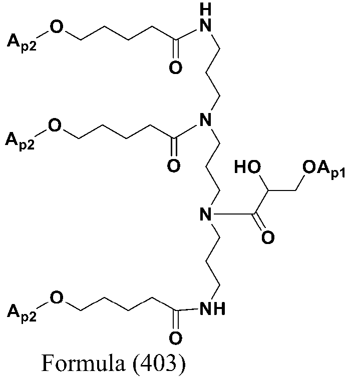 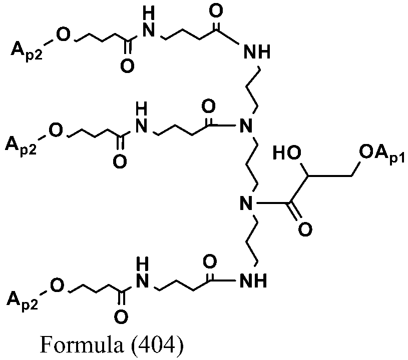 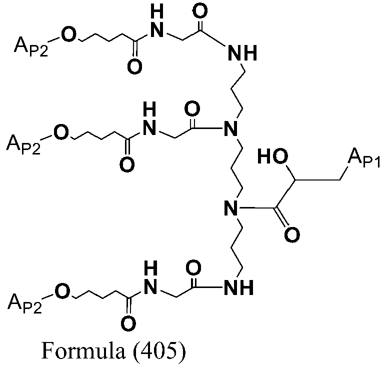 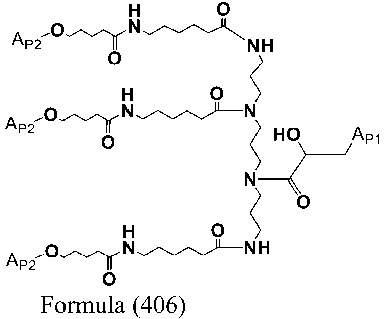 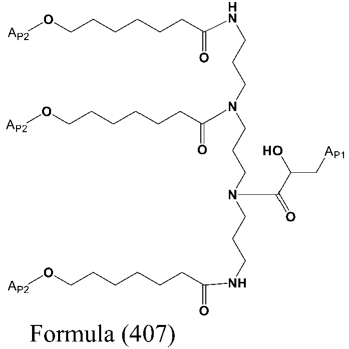 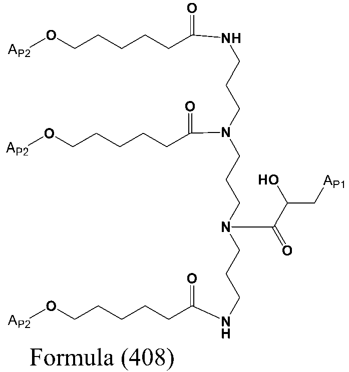 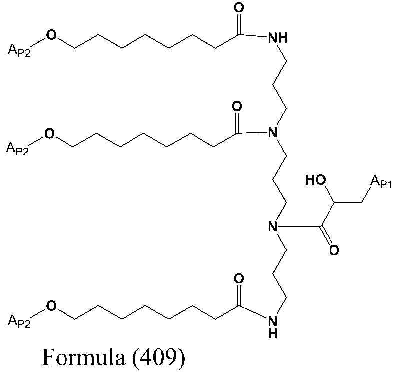 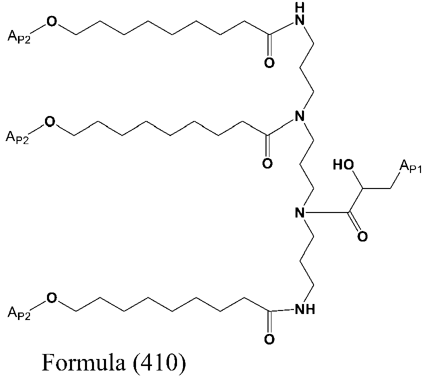 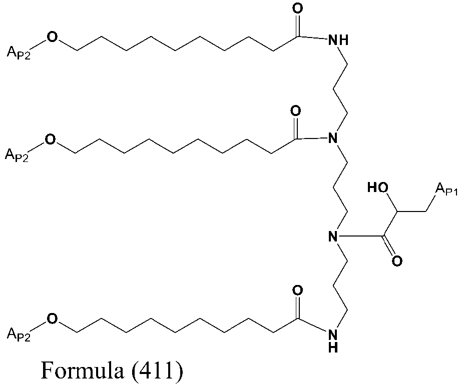 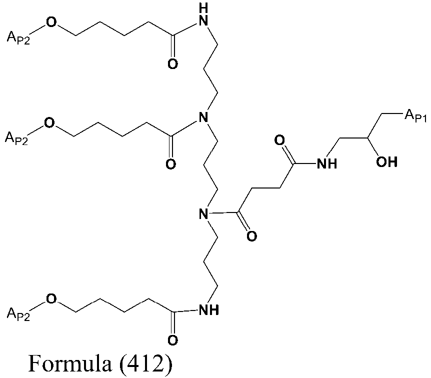 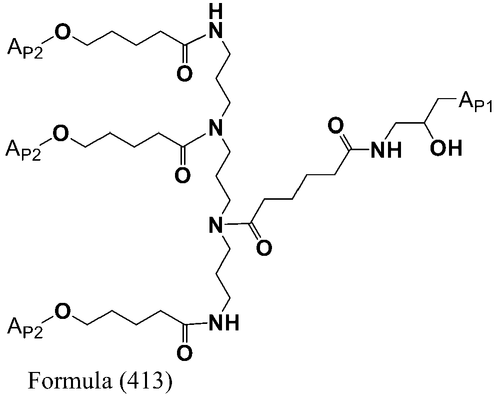 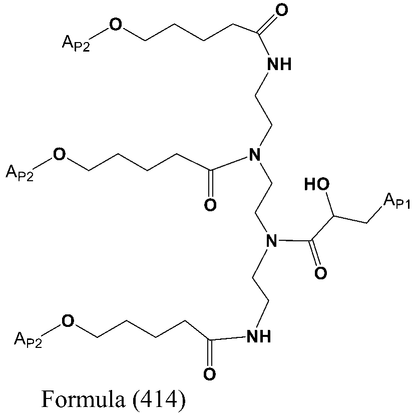 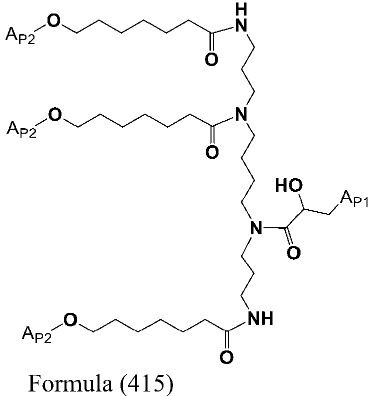 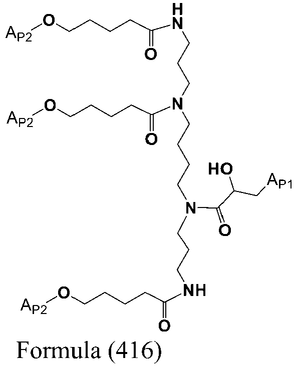 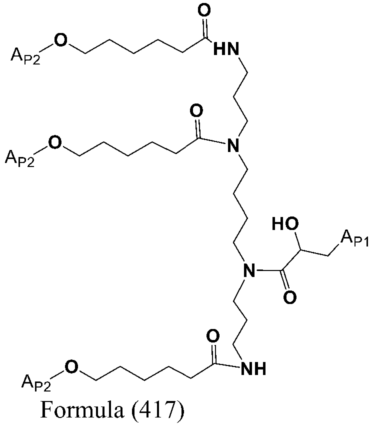 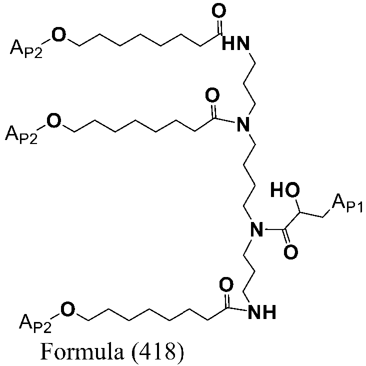 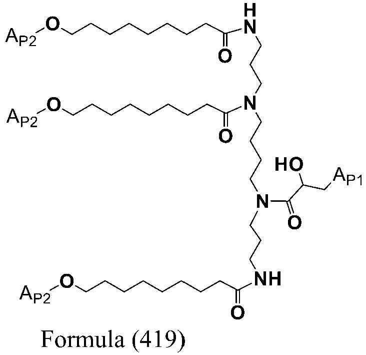 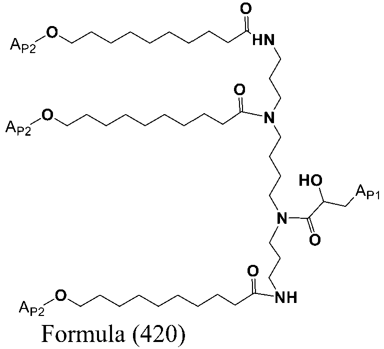 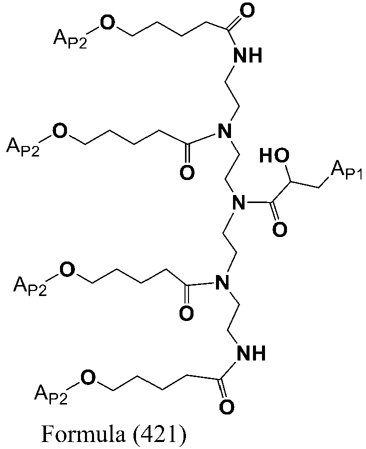 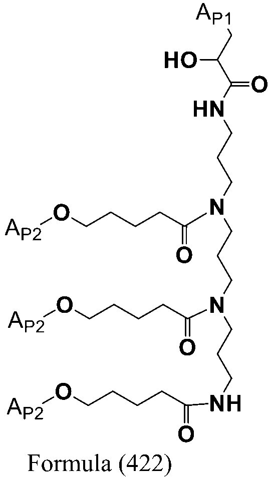 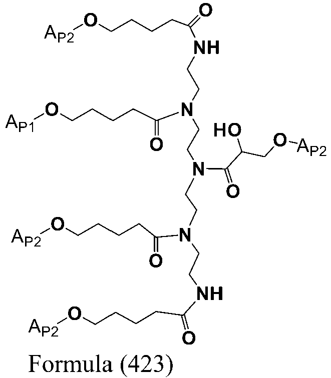 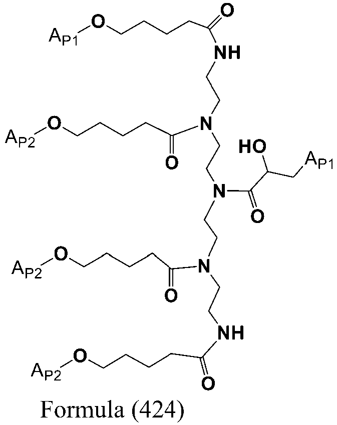 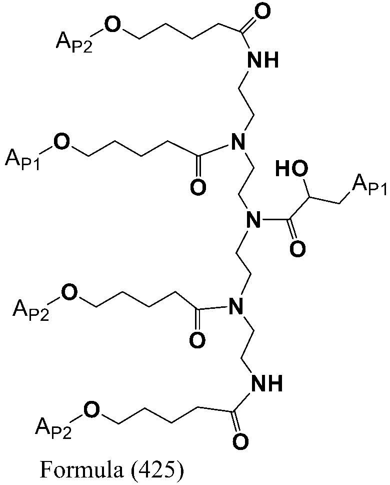 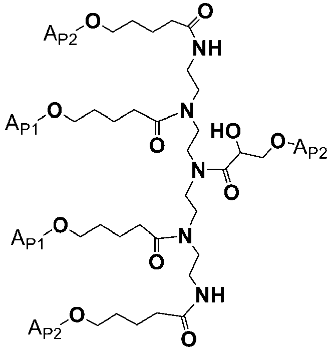 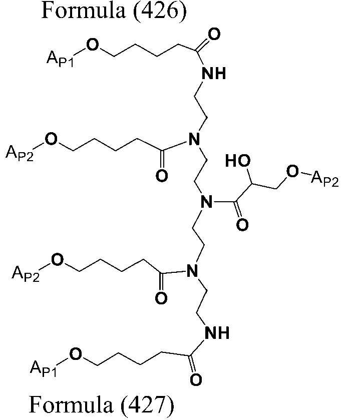 wherein A_{P1} is the chelating agent-derived group, and A_{P2} is the delivery group.

In some embodiments, the linking group Rⱼ comprises a nucleotide sequence I and a nucleotide sequence II, the nucleotide sequence I and the nucleotide sequence II each comprise 5-25 modified or unmodified nucleotides, and the nucleotide sequence I and the nucleotide sequence II are at least partly reverse complementary, the delivery group is linked to the nucleotide sequence I, the chelating agent-derived group is linked to the nucleotide sequence II, and the nucleotide sequence I and the nucleotide sequence II do not elicit immune response or toxic reaction in a subject. In some embodiments, the nucleotide sequence I and the nucleotide sequence II are substantially reverse complementary or completely reverse complementary; or the nucleotide sequence I and the nucleotide sequence II have the same length and are both 10-20 modified or unmodified nucleotides; or the nucleotide sequence I and the nucleotide sequence II both consist of 17 nucleotides and are completely reverse complementary to each other. In some embodiments, the 3' terminus of the delivery group is linked to the 5' position of the ribose in the 5'-terminal nucleotide of the nucleotide sequence I via a phosphate ester bond, and the chelating agent-derived group is linked to the 5' position of the ribose in the 5'-terminal nucleotide of the nucleotide sequence II; or the chelating agent-derived group comprises a segment of nucleotide sequence, and the 3' terminus of the nucleotide sequence is linked to the 5' position of the ribose in the 5'-terminus nucleotide of the nucleotide sequence I via a phosphate ester bond. In some embodiments, the nucleotide sequence I and the nucleotide sequence II respectively have the sequences as shown in SEQ ID NO: 40 and SEQ ID NO: 41:
5'-GUACAUUCUAGAUAGCC-3' (SEQ ID NO: 40)
5'-GGCUAUCUAGAAUGUAC-3' (SEQ ID NO: 41).

In some embodiments, the nucleotide sequence I and the nucleotide sequence II respectively have sequences as shown in SEQ ID NO: 42 and SEQ ID NO: 43:
5'-GmUfAmCfAmUfUfCfUfAmGmAmUfAmGmCfCf-3' (SEQ ID NO: 42)
5'-GmGmCfUfAmUfCfUfAmGmAmAmUfGmUfAmCf-3' (SEQ ID NO: 43)

In some embodiments, each of the chelating agent-derived groups is independently selected from the group consisting of the groups formed by removing one atom or functional group from DOTA, HBED-cc, NOTA, TRAP, TETA, and DiAmSar. Therein, the chelating agents DOTA, HBED-cc, NOTA, TRAP, TETA, and DiAmSar respectively have the following structures as shown in Formula (701) to Formula (706):

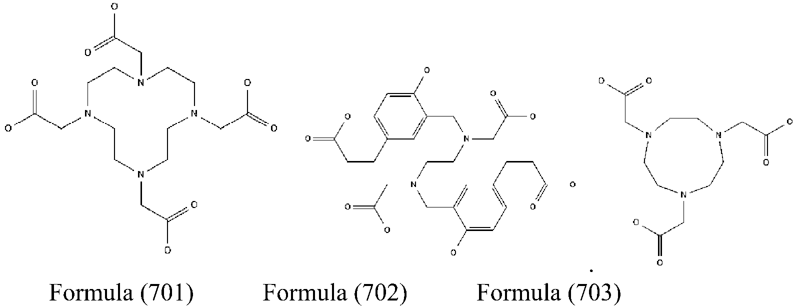

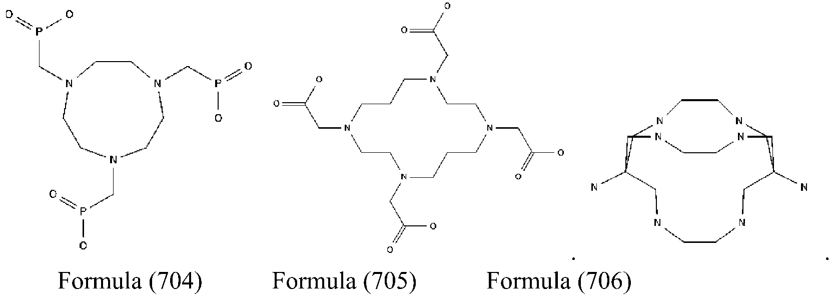

In some embodiments, each of the chelating agent-derived groups is independently a group formed by removing one atom or functional group from DOTA or NOTA. By comprising a chelating agent-derived group, the conjugate of the present disclosure can specifically deliver the chelating agent-derived group to tumors, and thus, through the effect of the chelating agent-derived group, diagnose and/or treat disease progression or symptoms of tumors.

In some embodiments, the conjugate provided by the present disclosure has the structure as shown in Formula (501), (502), or (504): 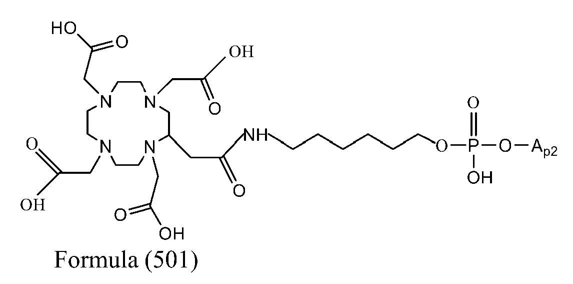 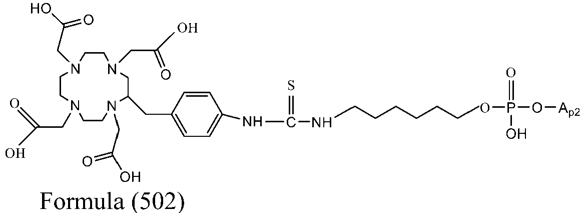 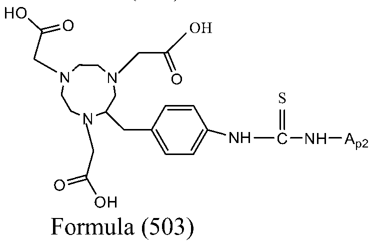 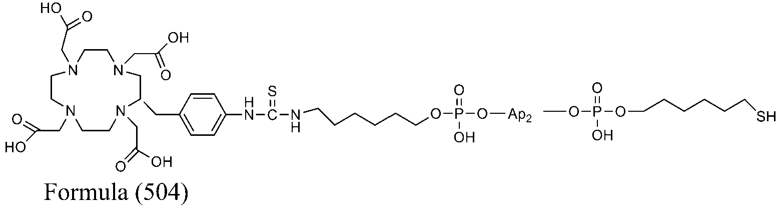 wherein the Aₚ₂ group is the delivery group.

In some embodiments, the present disclosure also provides a pharmaceutical conjugate comprising the conjugate provided by the present disclosure and a radionuclide, wherein the radionuclide and the chelating agent-derived group are linked via a coordinate bond.

In some embodiments, the radionuclide is at least one selected from the group consisting of ⁶⁸Ga, ¹⁷⁷Lu, ⁹⁰Y, ⁸⁹Zr, ^{99m}Tc, and ⁶⁴Cu. In some embodiments, the radionuclide is ⁶⁴Cu, ⁶⁸Ga, or ¹⁷⁷Lu.

In some embodiments, the pharmaceutical conjugate has the structure as shown in Formula (611), (612), (613), (614), or (615): 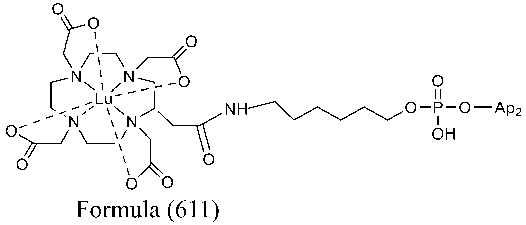 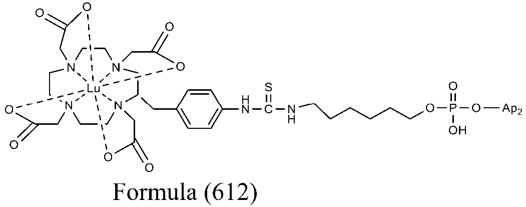 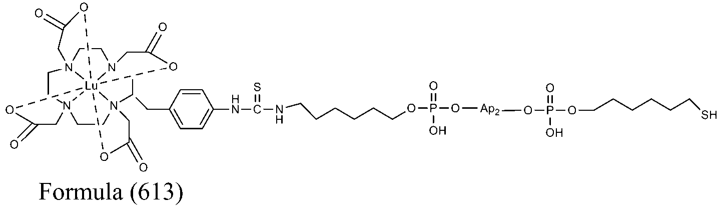 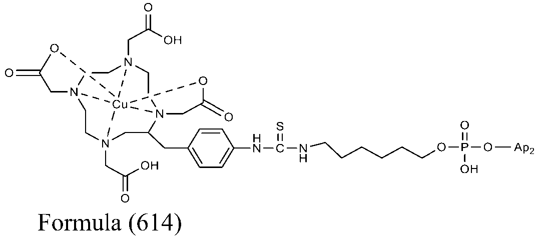 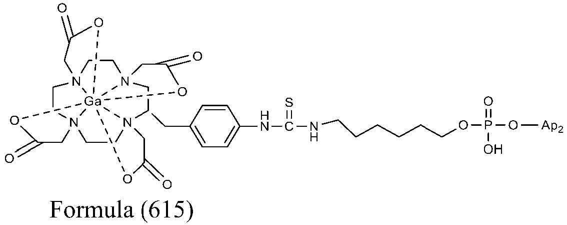 wherein the dashed lines represent coordinate bonds formed between the radionuclide and the chelating agent.

In some embodiments, the conjugate further comprises a diagnostic agent group, and the diagnostic agent group is linked to the delivery group via a covalent bond or via a linking group; and/or, the diagnostic agent group is linked to the chelating agent-derived group via a covalent bond or via a linking group.

In some embodiments, the conjugate comprises a diagnostic agent group, a chelating agent-derived group, and a delivery group, wherein the chelating agent-derived group, the delivery group, and the diagnostic agent group are sequentially linked via a covalent bond or via a linking group; or the chelating agent-derived group, the diagnostic agent group, and the delivery group are sequentially linked via a covalent bond or via a linking group.

In some embodiments, each of the diagnostic agent groups is independently selected from a contrast agent group or a fluorescence labelling tracer group. In some embodiments, the diagnostic agent group is a Cy5 fluorophore or a Cy3 fluorophore. When the conjugate provided by the present disclosure comprises different diagnostic agent groups and chelating agent-derived groups at the same time, it can simultaneously achieve the functions of diagnosis and treatment of diseases.

In some embodiments, the conjugate provided by the present disclosure further comprises one or more delivery aid groups, and the delivery aid group is one or more selected from the group consisting of C₁₀-C₃₀ hydrocarbyl, cholesteryl, and phospholipid groups. By comprising the delivery aid group, the conjugate provided by the present disclosure can be more compatible with the *in vivo* environment in the central nervous system, can have better bioavailability, and/or the conjugate provided by the present disclosure is delivered to tumors more effectively. In some embodiments, the delivery aid group is linked to the delivery group or the linking group via a covalent bond or via a linking group. In some embodiments, the delivery aid group is linked to the chelating agent-derived group.

Those skilled in the art can prepare the conjugate provided by the present disclosure using any appropriate synthetic route.

In some embodiments, the synthesis method of the conjugate provided by the present disclosure comprises contacting a protected conjugate with a deprotection reagent in a solvent under deprotection reaction condition, and isolating the conjugate provided by the present disclosure. The protected conjugate is a compound formed by protecting all active functional groups in the conjugate provided by the present disclosure with protecting groups. In some embodiments, the active functional groups include, but are not limited to, hydroxyl, amino, and/or phosphate group, and the protecting groups are correspondingly hydroxyl protecting groups, amino protecting groups, and/or phosphate hydroxyl protecting groups (e.g., cyanoethyl protecting groups). The solvent, deprotection reaction condition, and deprotection reagent to be used are selected and determined according to different protecting groups. In some embodiments, the deprotection reaction condition, solvent, and deprotection reagent are the deprotection reaction condition, solvent, and deprotection reagent used in solid phase synthesis of nucleic acids. In some embodiments, the method comprises adding the protected conjugate to a mixed solution of methylamine aqueous solution and aqueous ammonia, and the deprotection reaction condition comprises reacting for 1-5 h under normal temperature and pressure. In some embodiments, the methylamine aqueous solution and saturated concentrated aqueous ammonia are mixed in equal volumes to afford the mixed solution, whose amount is 0.1-10 mL/µmol relative to the protected conjugate. In some embodiments, the isolation comprises performing purification through column chromatography separation, collecting product eluate, and removing solvent. Purification condition can be, for example, performing elution by using a preparative ion chromatography purification column with a gradient eluant of sodium chloride solution and sodium phosphate solution. In some embodiments, gradient elution is performed with: 20 mM sodium phosphate (pH 8.1), solvent: water/acetonitrile in 9:1 (v/v); eluant B: 1.5 M sodium chloride, 20 mM sodium phosphate (pH 8.1), solvent: water/acetonitrile in 9:1 (v/v); elution gradient: the ratio of eluant A: eluant B = 100:0-50:50.

In some embodiments, the conjugate provided by the present disclosure has the structure as shown in Formula (101). The synthesis method of the protected conjugate comprises: contacting a compound comprising an active group Rₓ₁ and a delivery group with a compound comprising an active group Rₓ₂ and a chelating agent-derived group in an organic solvent under coupling reaction condition, and reacting to afford the protected conjugate, wherein the delivery group is formed by removing one or more hydrogen atoms or one or more functional groups from the aforementioned aptamer, wherein all active groups in the delivery group and the chelating agent-derived group are protected by protecting groups, and the active group Rₓ₁ and the active group R_{X2} are groups capable of reacting to form a covalent bond or linking group Rⱼ. In some embodiments, the molar ratio of the delivery group linked to an active group Rₓ₁ to the chelating agent-derived group linked to an active group Rₓ₂ is m₀:n₀. In some embodiments, active groups in the delivery group and chelating agent-derived group include, but are not limited to, one or more of hydroxyl, amino, and phosphate group, and the protecting group is correspondingly one or more selected from the group consisting of hydroxyl protecting group, amino protecting group, and phosphate hydroxyl protecting group (e.g. cyanoethyl protecting group).

Those skilled in the art could obtain the compound comprising an active group Rₓ₁ and a delivery group by various methods. In some embodiments, the compound comprising an active group Rₓ₁ and a delivery group can be obtained by nucleic acid synthesis methods well-known to those skilled in the art, for example, phosphoramidite solid phase synthesis or liquid phase synthesis by phosphodiester method/phosphotriester method. In some embodiments, the compound comprising an active group Rₓ₁ and a delivery group is obtained using the phosphoramidite solid phase synthesis method. The method comprises sequentially linking nucleoside monomers according to the sequence of the nucleotides in an oligonucleotide single strand under condition for phosphoramidite solid phase synthesis, wherein at least one nucleoside monomer is a nucleoside monomer having an active group Rₓ₁; or after linking all nucleoside monomers, linking a phosphoramidite monomer with an active group Rₓ₁ according to the phosphoramidite solid phase synthesis method, or linking a protected phosphoramidite monomer, and then removing the protecting group to form the active group Rₓ₁. The phosphoramidite solid phase synthesis method is well-known to those skilled in the art, whose process and conditions are disclosed in detail in Methods in Molecular Biology, vol. 288: Oligonucleotide Synthesis: Methods and Applications, P17-P31, which is incorporated herein by reference in its entirety.

In some embodiments, the coupling reaction condition is the condensation reaction condition or the condition for thiol-disulfide exchange reaction.

In some embodiments, the coupling reaction condition is the condensation reaction condition, which is acylation condensation reaction condition, dehydration condensation reaction condition, or click chemistry reaction condition, and the active group Rₓ₁ and active group Rₓ₂ are groups capable of undergoing the condensation reaction above. In some embodiments, the condensation reaction condition is acylation condensation reaction condition, and the active groups Rₓ₁ and Rₓ₂ are groups capable of undergoing acylation condensation reaction to form R_{I}. In some embodiments, the condensation reaction condition is dehydration condensation reaction condition, and one of the active groups Rₓ₁ and Rₓ₂ is a group comprising an acyl halide or carboxyl group, and the other is a group comprising an amino or hydroxyl group. In some embodiments, the condensation reaction condition is click chemistry reaction condition, and one of the active groups Rₓ₁ and Rₓ₂ is a group comprising an alkynyl group, and the other is a group comprising an azide group. In some embodiments, the condensation reaction condition is Michael addition reaction condition, and one of the active groups Rₓ₁ and Rₓ₂ is a group comprising a thiol group, and the other is a group comprising a succinimidyl group. In some embodiments, the condensation reaction condition is the condition for *N-*hydroxysuccinimide-carbodiimide (NHS-EDC) coupling reaction, and one of the active groups Rₓ₁ and Rₓ₂ is a group comprising N-hydroxysuccinimide (NHS) , and the other is a group comprising a carbodiimide (EDC).

In some embodiments, the compound comprising an active group Rₓ₁ and a delivery group is prepared by contacting the aptamer having an active group Rₓ₀ with a cross-linking agent under coupling reaction condition, and the cross-linking agent comprises a click chemistry active group and an acylation group. The active group Rₓ₀ and the acylation group are covalently linked through coupling reaction, linking the click chemistry active group to the aptamer of the present disclosure.

In some embodiments, an active group Rₓ₁ is an active group comprising 1-3 click chemistry active groups at its end, wherein the click chemistry active group comprises a terminal alkynyl group. In some embodiments, the acylation group is an active ester group, for example, one of an NHS ester group, an imidate group, and a pentafluorophenyl ester group. Those skilled in the art could obtain the cross-linking agent by various methods. For example, when the acylation group is a pentafluorophenyl ester group and the click chemistry group comprises a terminal alkynyl group, the cross-linking agent can be prepared according to the method as described in Scheme 1a (A) in Østergaard, Michael E., et al. "Efficient synthesis and biological evaluation of 5'-GalNAc conjugated antisense oligonucleotides." Bioconjugate chemistry 26.8 (2015): 1451-1455, which is incorporated herein by reference in its entirety. In some embodiments, the active group Rₓ₀ is an amino group. In some embodiments, the coupling condition is basic condition. In some embodiments, the basic condition is the condition in which a weak base aqueous solution is present, for example the condition in which a sodium bicarbonate aqueous solution is present.

Those skilled in the art could obtain the aptamer with an active group Rₓ₀ by various methods. In some embodiments, the aptamer with an active group Rₓ₀ is prepared by using a phosphoramidite monomer comprising an active group at the corresponding position during aptamer synthesis. Those skilled in the art could obtain a phosphoramidite monomer with an active group by various methods. In some embodiments, the active group Rₓ₀ is an amino group, and a phosphoramidite monomer with an Rₓ₀ can be commercially available or prepared by the methods well-known to those skilled in the art. For example, the phosphoramidite monomer with an Rₓ₀ can be readily commercially available 6-(trifluoroacetylamino)-hexyl-(2-cyanoethyl)-(N,N-diisopropyl)-phosphoramidite monomer, wherein the active group Rₓ₀ is an amino group. The active group Rₓ₀ can be obtained by linking the phosphoramidite monomer to an oligonucleotide single strand, and then removing the trifluoroacetyl protecting group through deprotection reaction readily realized by those skilled in the art (e.g., ammonolysis by concentrated aqueous ammonia) according to the phosphoramidite solid phase synthesis method.

In some embodiments, the coupling reaction condition is one of the conditions for thiol-disulfide exchange reaction, and one of the active groups Rₓ₁ and Rₓ₂ is a group comprising a thiol group, and the other comprises a leaving group linked by a disulfide bond. To avoid side reactions, in some embodiments, Rₓ₁ in the above phosphoramidite monomer with an active group Rₓ₁ is present in a form of protected Rₓ₁', and the preparation method further comprises the step of isolating the compound comprising an active group Rₓ₁ and a delivery group by contacting the prepared compound comprising the protected active group Rₓ₁" and a delivery group with a deprotection reagent under deprotection reaction condition. In some embodiments, the Rₓ₁' comprises a disulfide bond leaving group, the deprotection reaction condition is the condition for thiol-disulfide exchange reaction, and the deprotection reagent is a disulfide bond activator. In some embodiments, the disulfide bond activator is dithiodipyridine. Those skilled in the art could obtain the above phosphoramidite monomer with an active group Rₓ₁ or Rₓ₁' by various methods. In some embodiments, the phosphoramidite monomer with an active group Rₓ₁ or Rₓ₁' is commercially purchased. For example, the phosphoramidite monomer as shown in Formula (105) is commercially available. 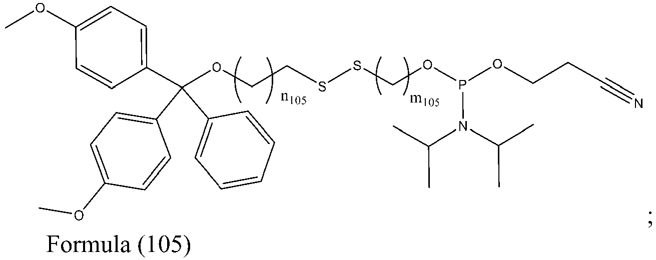 wherein n₁₀₅ and m₁₀₅ independently of one another are an integer of 1-10.

Those skilled in the art could obtain the compound comprising an active group Rₓ₂ and a chelating agent-derived group by various methods. In some embodiments, the coupling reaction condition is the condition for thiol-disulfide exchange reaction, the active group Rₓ₂ comprises a thiol group, and the compound comprising an active group Rₓ₂ and a chelating agent-derived group can be obtained by those skilled in the art via various known methods. For example, it can be prepared by using a phosphoramidite monomer comprising a thiol group according to the phosphoramidite solid phase synthesis method, or can be commercially purchased. In some embodiments, the coupling reaction condition is the condition for phosphoramidite solid phase synthesis reaction, the active group Rₓ₂ is a phosphoramidite group, and the compound comprising an active group Rₓ₂ and a chelating agent-derived group can be, for example, a readily commercially available compound comprising a phosphoramidite group and a chelating agent-derived group. In some embodiments, the coupling reaction condition is Michael addition reaction condition, the active group Rₓ₂ is a *N*-succinimidyl group, and the compound comprising an active group Rₓ₂ and a chelating agent-derived group can be, for example, a readily commercially available compound comprising a N-succinimidyl group and a chelating agent-derived group. In some embodiments, the active group Rₓ₁ and active group Rₓ₂ are respectively a nucleotide sequence I and a nucleotide sequence II, each of which comprises 5-25 modified or unmodified nucleotides, and the nucleotide sequence I and the nucleotide sequence II are at least partly reverse complementary. The delivery group is linked to the nucleotide sequence I, the chelating agent-derived group is linked to the nucleotide sequence II, and the nucleotide sequence I and the nucleotide sequence II do not elicit immune response or toxic reaction in a subject. The coupling reaction condition is the reaction condition for annealing to form a nucleic acid double strand. In some embodiments, the nucleotide sequence I and the nucleotide sequence II both consist of 17 nucleotides and are completely reverse complementary to each other. In some embodiments, the nucleotide sequence I and the nucleotide sequence II respectively have the sequences as shown in SEQ ID NO: 40 and SEQ ID NO: 41. In some embodiments, the nucleotide sequence I and the nucleotide sequence II respectively have the sequences as shown in SEQ ID NO: 42 and SEQ ID NO: 43.

The delivery group is formed by removing one or more hydrogen atoms or functional groups from an aptamer. In some embodiments, the group at the 5'position of the ribose in the 5'-terminal nucleotide and the group at the 3'position of the ribose in the 3'-terminal nucleotide of the consecutive nucleotide sequence are both hydroxyl groups, and the delivery group is formed by removing one hydrogen atom from the 5'-hydroxyl group of the 5'-terminal nucleotide of the aptamer. In some embodiments, the delivery group is formed by removing one hydrogen atom from the 3'-hydroxyl group of the 3'-terminal nucleotide of the aptamer. In some embodiments, the delivery group is formed by removing the 5'-hydroxyl group from the 5'-terminal nucleotide of the aptamer. In some embodiments, the delivery group is formed by removing the 3'-hydroxyl group from the 3'-terminal nucleotide of the aptamer. In some embodiments, the delivery group is formed by removing the 2'-hydroxyl group of the ribose from the nucleotide contained in the aptamer. The aptamer can be obtained by the conventional method for preparing an aptamer in the art (e.g., solid phase synthesis and liquid phase synthesis methods of nucleic acids), wherein the solid-phase synthesis of nucleic acids already has commercial customized services. A modified nucleotide can be introduced into the conjugate provided by the present disclosure by using a nucleoside monomer with a corresponding modification. The methods for preparing a nucleoside monomer with a corresponding modification and the methods for introducing a modified nucleotide into the aptamers are also well known to those skilled in the art. All nucleoside monomers with modifications can be commercially available or prepared by known methods.

In some embodiments, the conjugate can also be used in the present disclosure in the form of a pharmaceutically acceptable salt or a precursor compound thereof. In the context of the present disclosure, a "pharmaceutically acceptable salt" refers to a corresponding salt formed by a drug to increase the stability, solubility, and/or bioavailability of the drug without pharmaceutically producing additional side effects on the human body, such as potassium salt, sodium salt, or carboxylate. A "precursor compound" refers to a compound that is not identical with the conjugate in terms of structure and function, but could react and form the conjugate provided by the present disclosure after entering into the body or in the body fluid environment, thereby exerting its effect and achieving the purpose of the present disclosure. Under certain circumstances, these precursor compounds can increase the stability, extend the slow-release time, increase the bioavailability, etc. of the drug. In some embodiments, the precursor compound comprises the precursor groups capable of reacting in the human body to form all chelating agent-derived groups in a conjugate. In some embodiments, the precursor compound includes a compound formed by substituting all active hydroxyl groups in a conjugate with acetoxy groups. In some embodiments, the precursor compound comprises a drug precursor group, which is a residue formed by a precursor compound corresponding to the chelating agent-derived group in a conjugate. In some embodiments, the drug precursor group can be, for example, a group formed by substituting active hydrogen in a hydroxyl or amino functional group in the chelating agent-derived group with an acyl, alkyl, or phosphoryl group. Those skilled in the art could understand that use of these pharmaceutically acceptable salts and precursor compounds are also within the scope of the present disclosure.

In some embodiments, in the conjugate of the present disclosure, each pair of adjacent nucleotides is linked via a phosphodiester bond or phosphorothioate diester bond, wherein the non-bridging oxygen atom or sulfur atom in the phosphodiester bond or phosphorothioate diester bond bear negative charges, and can be present in the form of hydroxy or thiol, and wherein some or all hydrogen ions in the hydroxy or thiol can be substituted with cation(s). The cation can be any cation, such as one of a metal cation, an ammonium ion NH₄⁺ or an organic ammonium cation. In order to increase solubility and/or improve bioavailability, in some embodiments, the pharmaceutically acceptable salt is a partial or completely water-soluble salt of the conjugate. In some embodiments, the water-soluble salt can be an amine salt, an alkali metal salt, or an alkaline earth metal salt. In some embodiments, the amine salt is one or more selected from the group consisting of an ammonium salt, a methylamine salt, a tertiary amine salt, and a quaternary ammonium salt; the alkali metal salt is selected from potassium salts or sodium salts; and the alkaline earth metal salt is selected from calcium salts or magnesium salts. In some embodiments, the tertiary amine salt is a triethylamine salt, a triisopropylamine salt, or an N,N-diisopropylethylamine salt. In some embodiments, the pharmaceutically acceptable salt of the conjugate is a mixture of a methylamine salt and an ammonium salt of the conjugate. In some embodiments, the pharmaceutically acceptable salt of the conjugate is a sodium salt or a partial sodium salt of the conjugate.

### Pharmaceutical composition

In one aspect, the present disclosure further provides a pharmaceutical composition, comprising the pharmaceutical conjugate provided by the present disclosure and a pharmaceutically acceptable carrier.

The pharmaceutically acceptable carrier is one or more of various conventional ingredients in the art, such as, one or more of a solvent, a protectant, an osmotic pressure regulator, and other pharmaceutically acceptable carriers.

For example, when the pharmaceutical composition is an injection solution, the pharmaceutically acceptable excipient is a solvent, such as, deionized water, water for injection, ethanol, and pH buffer. The pH buffer can be a tris(hydroxymethyl) aminomethane hydrochloride buffer with a pH of 7.5-8.5, and/or a phosphate buffer with a pH of 5.5-8.5, such as a phosphate buffer with a pH of 5.5-8.5.

The dosage of the solvent is adjusted according to the required solution concentration, and the concentration of the conjugate in the injection solution can be 0.01 mg/mL-20 mg/mL, 0.1 mg/mL-10 mg/mL or 0.5 mg/mL-5 mg/mL, as calculated based on the nucleotide sequence group in the conjugate.

Other pharmaceutically acceptable carrier can be a carrier conventionally used in the art, for example, but not limited to, magnetic nanoparticles (such as Fe₃O₄-based or Fe₂O₃-based nanoparticles), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, the pharmaceutically acceptable carrier comprises a physiologically acceptable compound, which exerts the function of, for example, stabilizing the pharmaceutical composition or increasing or decreasing the absorption of the conjugate and/or the pharmaceutical composition. The physiologically acceptable compound is one or more selected from the following compounds: carbohydrates, such as glucose, sucrose, and/or glucan; antioxidants, such as ascorbic acid and/or glutathione; low molecular weight proteins; compositions that reduce the clearance or hydrolysis of any co-administered substance; excipients; stabilizers and buffers. A detergent can also be used for stabilizing the composition or increasing or decreasing the absorption of the pharmaceutical composition. The physiologically acceptable compound can further include one or more of wetting agents, emulsifiers, dispersants, or preservatives especially used for preventing microbial growth or action. The physiologically acceptable compound is known to those skilled in the art, which is not described here in detail. Those skilled in the art could easily understand that the choices of the pharmaceutically acceptable carrier and physiologically acceptable compound depend on, for example, administration routes and specific physiochemical characteristics of any co-administered substance.

In some embodiments, the pharmaceutically acceptable carrier is sterile and usually does not comprise an undesirable substance. The pharmaceutical composition provided by the present disclosure can further comprise pharmaceutically acceptable auxiliary substances according to requirements to approach physiological conditions, such as pH regulators and buffers and toxicity regulators, for example sodium acetate, sodium chloride, potassium chloride, calcium chloride, and sodium lactate. The concentration of the pharmaceutical conjugate provided by the present disclosure in the pharmaceutical composition can vary within a wide range, and is selected mainly according to fluid volume, viscosity, body weight, etc. and according to the specific administration manner.

In some embodiments, in the pharmaceutical composition, there are no special requirements for the contents of the pharmaceutical conjugate and the pharmaceutically acceptable carrier. In some embodiments, the weight ratio of the pharmaceutical conjugate to the pharmaceutically acceptable carrier can be 1:(1-500), and in some embodiments, the above weight ratio is 1:(1-50).

In some embodiments, the carrier can be selected from osmotic pressure regulators, and the osmotic pressure regulator can be sodium chloride and/or potassium chloride. The content of the osmotic pressure regulator allows the osmotic pressure of the pharmaceutical composition to be 200-700 mOsm/kg. Depending on the desired osmotic pressure, those skilled in the art can readily determine the content of the osmotic pressure regulator. In some embodiments, the dose of the formulation prepared from the pharmaceutical composition will be adjusted according to different administration manners during administration.

In some embodiments, the carrier can be selected from protectants, and the protectant can be at least one of inositol, sorbitol, sucrose, trehalose, mannose, maltose, lactose, and glucose. The content of the protection agent can be from 0.01 wt% to 30 wt% based on the total weight of the pharmaceutical composition.

In some embodiments, the pharmaceutical composition can be a liquid formulation, for example, an injection solution; or a lyophilized powder for injection, which will be mixed with a liquid adjuvant to form a liquid formulation upon administration. The liquid formulation can be used for, but not limited to, administration by subcutaneous, intramuscular, or intravenous injection, and the pharmaceutical composition can also be delivered by, but not limited to, puncture injection, oropharyngeal inhalation, nasal administration, or other routes. In some embodiments, the pharmaceutical composition is used for administration by subcutaneous, intramuscular, intravenous, or intrathecal injection.

In some embodiments, the pharmaceutical composition can be in the form of a liposome formulation. In some embodiments, the pharmaceutically acceptable carrier used in the liposome formulation comprises an amine-containing transfection compound (hereinafter also referred to as a key lipid), a helper lipid, and/or a PEGylated lipid. Therein, the key lipid, the helper lipid, and the PEGylated lipid can be respectively selected from one or more of the amine-containing transfection compounds or the pharmaceutically acceptable salts or derivatives thereof, the helper lipids, and the PEGylated lipids as described in the Chinese patent application CN103380113A, which is incorporated herein by reference in its entirety.

In some embodiments, the key lipid can be the compound as shown in Formula (201) or a pharmaceutically acceptable salt thereof as described in the Chinese patent application CN103380113A: 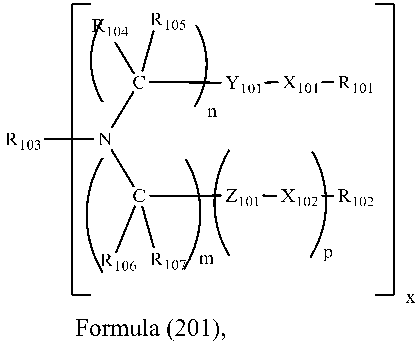 wherein:
X₁₀₁ and X₁₀₂ independently of one another are O, S, N-A or C-A, wherein A is hydrogen or a C₁-C₂₀ hydrocarbon chain;
Y₁₀₁ and Z₁₀₁ independently of one another are C=O, C=S, S=O, CH-OH or SO₂;
R₁₀₁, R₁₀₂, R₁₀₃, R₁₀₄, R₁₀₅, R₁₀₆, and R₁₀₇ independently of one another are hydrogen;
a cyclic or an acyclic, substituted or unsubstituted, branched or straight-chain aliphatic group; a cyclic or an acyclic, substituted or unsubstituted, branched or straight-chain heteroaliphatic group; a substituted or unsubstituted, branched or straight-chain acyl group; a substituted or unsubstituted, branched or straight-chain aryl group; or a substituted or unsubstituted, branched or straight-chain heteroaryl group;
x is an integer of 1-10;
n is an integer of 1-3, m is an integer of 0-20, and p is 0 or 1, wherein if m = p = 0, then R₁₀₂ is hydrogen; and
if at least one of n and m is 2, then R₁₀₃ and the nitrogen in Formula (201) form the structure as shown in Formula (202) or (203): 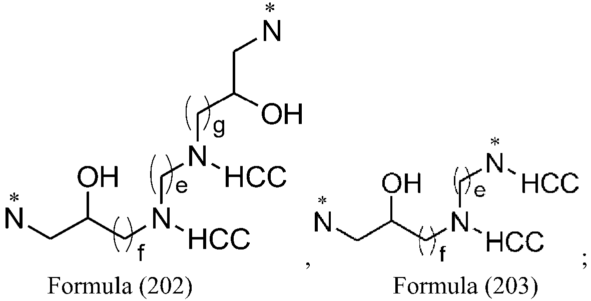
wherein g, e, and f independently of one another are an integer of 1-6, "HCC" represents a hydrocarbon chain, and each *N represents a nitrogen atom in Formula

(201). In some embodiments, R₁₀₃ is a polyamine. In other embodiments, R₁₀₃ is a ketal. In some embodiments, R₁₀₁ and R₁₀₂ in Formula (201) independently of one another are any substituted or unsubstituted, branched or straight-chain alkyl or alkenyl, wherein the alkyl or alkenyl has 3 to about 20 carbon atoms (such as 8 to about 18 carbon atoms) and 0-4 double bonds (such as 0-2 double bonds).

In some embodiments, if n and m independently of one another are 1 or 3, R₁₀₃ can be any of the following Formulae (204)-(213): 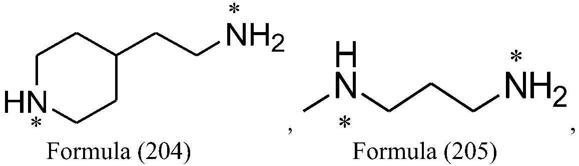 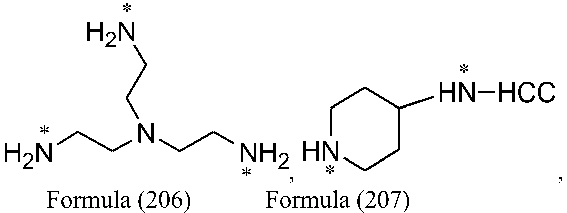 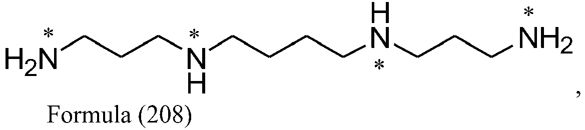 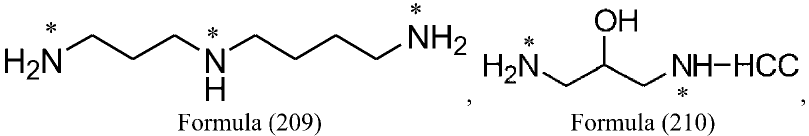 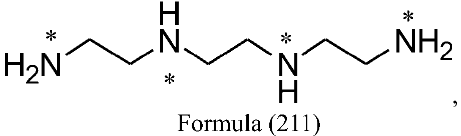 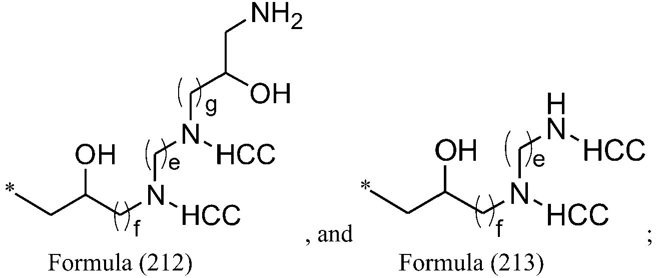 wherein in Formulae (204)-(213), g, e, and f independently of one another are an integer of 1-6, each "HCC" represents a hydrocarbon chain, and each * represents a potential attachment point of R₁₀₃ to the nitrogen atom in Formula (201), wherein each H at any * position can be replaced to achieve the attachment to the nitrogen atom in Formula (201).

Those skilled in the art could obtain the compound as shown in Formula (201) by any appropriate method. In some embodiments, the compound as shown in Formula (201) can be prepared according to the description of the Chinese patent application CN103380113A.

In some embodiments, the key lipid is the key lipid as shown in Formula (214) and/or the key lipid as shown in Formula (215):

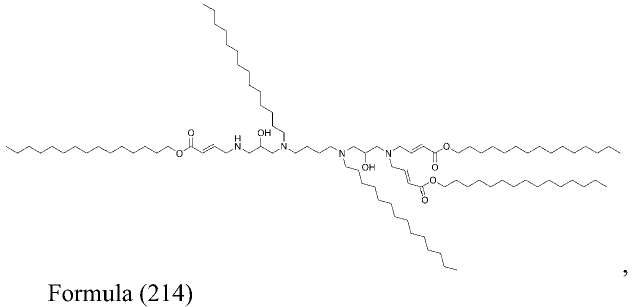

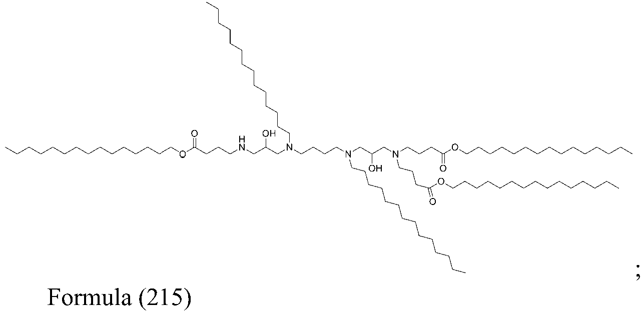

The helper lipid is cholesterol, a cholesterol analog, and/or a cholesterol derivative.

The PEGylated lipid is 1,2-dipalmitoyl-sn-glycero-3-phosphoethanolamine-N-[methoxy(polyethylene glycol)]-2000.

In some embodiments, the molar ratio among the key lipid, the helper lipid, and the PEGylated lipid in the pharmaceutical composition is (19.7-80):(19.7-80):(0.3-50); for example, the molar ratio can be (50-70):(20-40):(3-20).

In some embodiments, the pharmaceutical composition particles formed by the conjugate provided by the present disclosure and the above amine-containing transfection reagents have an average diameter of about 30 nm to about 200 nm, typically about 40 nm to about 135 nm, and more typically, the average diameter of the liposome particles is about 50 nm to about 120 nm, about 50 nm to about 100 nm, about 60 nm to about 90 nm, or about 70 nm to about 90 nm; for example, the average diameter of the liposome particles is about 30, 40, 50, 60, 70, 75, 80, 85, 90, 100, 110, 120, 130, 140, 150, or 160 nm.

In some embodiments, in the pharmaceutical composition formed by the conjugate provided by the present disclosure and the above amine-containing transfection reagents, the weight ratio (weight/weight ratio) of the conjugate to total lipids, e.g., the key lipids, the helper lipids, and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the conjugate provided by the present disclosure to total lipids is about 1:5, 1:6, 1:7, 1:8, 1:9, 1:10, 1:11, 1:12, 1:13, 1:14, 1:15, 1:16, 1:17, or 1:18.

In some embodiments, the pharmaceutical composition can be marketed with each component being separate, and used in the form of a liquid formulation. In some embodiments, the pharmaceutical composition formed by the conjugate provided by the present disclosure and the above pharmaceutically acceptable carrier can be prepared by various known processes, except for replacing the existing aptamer or conjugate with the conjugate provided by the present disclosure. In some embodiments, the pharmaceutical composition can be prepared according to the following process:
The key lipids, helper lipids, and PEGylated lipids are suspended in alcohol at a molar ratio above and mixed homogeneously to yield a lipid solution; the alcohol is used in an amount such that the resultant lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, polyethylene glycol 200, polyethylene glycol 300, and polyethylene glycol 400, such as ethanol.

The conjugate provided by the present disclosure is dissolved in a buffered salt solution to produce an aqueous solution of the conjugate. The buffered salt solution has a concentration of 0.05 to 0.5 M, such as 0.1 to 0.2 M. The pH of the buffered salt solution is adjusted to 4.0 to 5.5, such as 5.0 to 5.2. The buffered salt solution is used in an amount such that the conjugate is present at a concentration of no more than 0.6 mg/mL, such as 0.2 to 0.4 mg/mL. The buffered salt can be one or more selected from the group consisting of soluble acetates and soluble citrates, such as sodium acetate and/or potassium acetate.

The lipid solution and the aqueous solution of the conjugate are mixed. The product obtained by mixing is incubated at a temperature of 40 to 60°C for at least 2 minutes (e.g., 5 to 30 minutes) to produce an incubated liposome formulation. The volume ratio of the lipid solution to the aqueous solution of the conjugate is 1:(2-5), such as 1:4.

The incubated liposome formulation is concentrated or diluted, subjected to impurity removal, and then sterilized to afford the pharmaceutical composition provided by the present disclosure, which has the following physicochemical parameters: a pH of 6.5 to 8, an encapsulation efficiency of no less than 80%, a particle size of 40 to 200 nm, a polydispersity index of no more than 0.30, and an osmotic pressure of 250 to 400 mOsm/kg. For example, the physicochemical parameters can be as follows: a pH of 7.2 to 7.6, an encapsulation efficiency of no less than 90%, a particle size of 60 to 100 nm, a polydispersity index of no more than 0.20, and an osmotic pressure of 300 to 400 mOsm/kg.

Therein, the concentration or dilution step can be performed before, after, or simultaneously with removal of the impurities. The method for removing impurities can be any of various existing methods, for example, ultrafiltration using a tangential flow system and a hollow fiber column under 100 kDa, with a phosphate buffer (PBS) at pH 7.4 as ultrafiltration exchange solution. The method for sterilization can be any of various existing methods, such as filtration sterilization on a 0.22 µm filter.

### Use of the conjugate of the present disclosure

In another aspect, the present disclosure further provides use of the pharmaceutical conjugate and/or pharmaceutical composition provided by the present disclosure in the manufacture of a medicament for diagnosing and/or treating tumors and tumor-related diseases or symptoms. In some embodiments, the tumors in the present disclosure are one or more of glioma, renal cancer, and lung carcinoma.

In yet another aspect, the present disclosure further provides a method for diagnosing and/or treating tumors and tumor-related diseases or symptoms, comprising administering the pharmaceutical conjugate and/or the pharmaceutical composition provided by the present disclosure to a subject in need thereof. In some embodiments, the tumors in the present disclosure are one or more of glioma, renal cancer, and lung carcinoma.

By administering the pharmaceutical conjugate and/or the pharmaceutical composition provided by the present disclosure, the method of the present disclosure can effectively treat tumors and tumor-related diseases or symptoms; and with the highly specific targeting effect of the conjugate provided by the present disclosure, the distribution of the therapeutic agent in other undesired organs/tissues of the body can be decreased, thereby reducing potential side effects, which is of great importance and significant value especially for radiotherapy and/or chemotherapy drugs commonly used in the field of tumor treatment and known for significant side effects.

As used herein, the term "administration/administer" refers to placing the pharmaceutical conjugate and/or the pharmaceutical composition into a subject by a method or route where the pharmaceutical conjugate and/or the pharmaceutical composition is at least partly located at a desired site to achieve a desired effect. The administration routes suitable for the method of the present disclosure include topical administration and systemic administration. In general, topical administration results in the delivery of more of the pharmaceutical conjugate and/or the pharmaceutical composition to a particular site as compared with the whole body of the subject; whereas systemic administration results in the delivery of the pharmaceutical conjugate and/or the pharmaceutical composition to substantially the whole body of the subject.

Furthermore, the inventors of the present disclosure have unexpectedly found that the pharmaceutical conjugate and/or the pharmaceutical composition of the present disclosure can efficiently pass through the blood brain barrier and target tumors in the brain upon systemic administration, thereby further improving the delivery efficiency of the functional groups, saving costs, and reducing undesired side effects.

The administration to a subject can be achieved by any suitable route known in the art, including but not limited to, oral or parenteral routes, such as intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency can be once or more times daily, weekly, biweekly, triweekly, monthly, or yearly.

The dose of the pharmaceutical conjugate and/or the pharmaceutical composition of the present disclosure can be a conventional dose in the art, which can be determined according to various parameters, especially age, weight, and gender of a subject. Toxicity and efficacy can be determined in cell cultures or experimental animals by standard pharmaceutical procedures, for example, by determining LD50 (the lethal dose that causes 50% population death) and ED50 (the dose that can cause 50% of the maximum response intensity in a quantitative response, or that causes 50% of the experimental subjects to have a positive response in a qualitative response). The dose range for human use can be derived based on the data obtained from cell culture analysis and animal studies.

When the pharmaceutical conjugate and/or the pharmaceutical composition of the present disclosure is administered, for example, to male or female C57BL/6J or C3H/HeNCrlVr mice with an age of 6 to 12 weeks and a body weight of 18 to 25 g (based on the amount of the pharmaceutical conjugate and/or the conjugate in the pharmaceutical composition): the dosage of the conjugate can be 0.001 to 100 mg/kg body weight, in some embodiments 0.01 to 50 mg/kg body weight, in some further embodiments 0.05 to 20 mg/kg body weight, in some even further embodiments 0.1 to 15 mg/kg body weight, and in some still further embodiments 0.1 to 10 mg/kg body weight. When the conjugate and/or the pharmaceutical composition of the present disclosure is administered, the above dosages can be preferred.

### Kit

The present disclosure provides a kit comprising the pharmaceutical conjugate and/or the pharmaceutical composition provided by the present disclosure.

In some embodiments, the kit of the present disclosure can provide the pharmaceutical conjugate and/or the pharmaceutical composition in a container. In some embodiments, the kit of the present disclosure can comprise a container for providing pharmaceutically acceptable excipients. In some embodiments, the kit can further comprise additional ingredients, such as stabilizers or preservatives. In some embodiments, the kit of the present disclosure can comprise at least one additional therapeutic agent in a container other than the container for providing the pharmaceutical conjugate and/or the pharmaceutical composition of the present disclosure. In some embodiments, the kit can comprise an instruction for mixing the pharmaceutical conjugate and/or the pharmaceutical composition with pharmaceutically acceptable carriers and/or adjuvants or other ingredients (if any).

In the kit of the present disclosure, the pharmaceutical conjugate and the pharmaceutically acceptable carriers and/or adjuvants, as well as the pharmaceutical composition and/or the pharmaceutically acceptable adjuvants can be provided in any form, e.g., in a liquid form, a dry form, or a lyophilized form. In some embodiments, the pharmaceutical conjugate and the pharmaceutically acceptable carriers and/or adjuvants as well as the pharmaceutical composition and the optional pharmaceutically acceptable adjuvants are substantially pure and/or sterile. In some embodiments, the kit of the present disclosure can provide sterile water.

Hereinafter, the present disclosure will be further illustrated with reference to the Examples, but is not limited thereto in any respect.

### Examples

Unless otherwise specified, the reagents and culture media used in the following Examples are all commercially available, and the procedures used, such as nucleic acid electrophoresis and real-time PCR, are all performed according to the methods described in Molecular Cloning (Cold Spring Harbor Laboratory Press (1989)).

For the conjugates synthesized and used in the following Examples, unless otherwise specified, all the conjugates obtained are the sodium salts of the conjugates in which the hydroxyl hydrogen ions in all phosphate groups are substituted by sodium ions.

### Preparation Example 1 Synthesis of Conjugate RA1

In this Preparation Example, Conjugate RA1 was prepared according to the following preparation procedure:

### (1) Synthesis of a nucleotide sequence

Through the nucleic acid solid phase synthesis method (phosphoramidite solid phase method), nucleoside monomers were linked one by one in a 3'-5' direction according to the sequence as shown in SEQ ID NO: 24 to afford the following nucleotide sequence:

5'-CmUmGmGmAmGmUmUmCmAmGmAmCmGmUmUmGmUmGmCmUmCmU
mUm-3' (SEQ ID NO: 24)

Therein, C, G, U, A, and T represent the base composition of nucleotide; m represents that the nucleotide adjacent to the left side of the letter m is a methoxy modified nucleotide.

Through the phosphoramidite solid phase synthesis method, nucleoside monomers were linked one by one in a 3'-5' direction according to the arrangement sequence of nucleotides in the sense strand. The linking of each nucleoside monomer includes a four-step reaction of deprotection, coupling, capping, and oxidation. When two nucleotides are linked via a phosphoester linkage, a four-step reaction of deprotection, coupling, capping, and oxidation is used for linking the later nucleoside monomer. The synthesis conditions are given as follows.

The nucleoside monomers were provided in a 0.1 M acetonitrile solution. The condition for deprotection reaction in each step is identical, i.e., a temperature of 25°C, a reaction time of 70 seconds; a solution of dichloroacetic acid in dichloromethane (3% v/v) as a deprotection reagent; and a molar ratio of dichloroacetic acid to the protecting group 4,4'-dimethoxytrityl on the solid phase support of 5:1.

The condition for coupling reaction in each step is identical, including: a temperature of 25°C; a molar ratio of the nucleic acid sequence linked to the solid phase support to nucleoside monomers of 1:10; a molar ratio of the nucleic acid sequence linked to the solid phase support to a coupling reagent of 1:65; a reaction time of 600 seconds; and 0.5 M acetonitrile solution of 5-ethylthio-1H-tetrazole (ETT) as a coupling reagent.

The condition for capping reaction in each step is identical, including a temperature of 25°C and a reaction time of 15 seconds; a mixed solution of Cap A and Cap B in a molar ratio of 1:1 as a solution of capping agent; and a molar ratio of the capping agent to the nucleic acid sequence linked to the solid phase support of 1:1:1 (acetic anhydride: N-methylimidazole: the nucleic acid sequence linked to the solid phase support).

The condition for oxidation reaction in each step is identical, including: a temperature of 25°C and a reaction time of 15 seconds; 0.05 M iodine water as an oxidation reagent; and a molar ratio of iodine to the nucleic acid sequence linked to the solid phase support in the coupling step of 30:1. The reaction was carried out in a mixed solvent of tetrahydrofuran: water: pyridine = 3:1:1.

(2) During the solid phase synthesis, in a 3'-5' direction, after linking the last nucleoside monomer at the 5' terminus, an additional phosphoramidite monomer as shown in Formula (110) was linked according to the solid phase synthesis method to afford the nucleotide sequence linked to a protected amino terminus 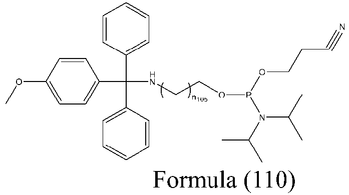 wherein n₁₀₅ is 5, and the reaction conditions for deprotection, coupling, capping, and oxidation used in the linking method were identical with the reaction conditions above.

The nucleotide sequence (20 mg, 1.0 eq) linked to an alkynyl terminus obtained in the above process was added into 25 wt% aqueous ammonia to react at 55°C for 16 hours, wherein the amount of aqueous ammonia used is 0.5 mL/ µ mol). After removing the liquid, the mixture was concentrated to dryness under vacuum to afford the nucleotide sequence linked to an amino terminus as shown in Formula (120):

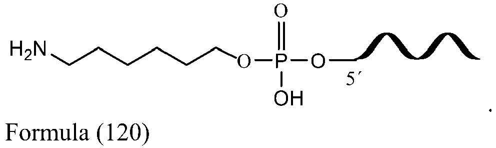

The compound (7 mg, 1.0 eq) as shown in Formula (120), obtained after ultrafiltration, sodium salt conversion, and concentration, was dissolved in 0.175 mL of a sodium bicarbonate aqueous solution (100 Mm) to afford a solution of the nucleotides linked to an amino terminus.

The compound (3 mg, 8.0 eq) as shown in Formula (130) was dissolved in 0.7 mL of DMF; the resultant solution was mixed with the nucleotide solution obtained in the above step; and the mixed solution was added with 14 µL of triethylamine, stirred thoroughly, and reacted at room temperature for 2 h to afford the crude reaction product.

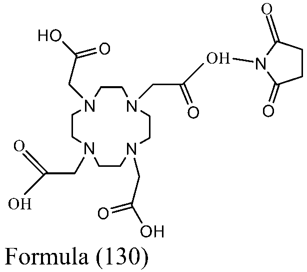

(3) 10 µL of the crude reaction product was mixed with 90 µL of a mixed solvent (consisting of DMF and water in a volume ratio of 1:1) to afford a solution of the crude reaction product. After the solution was vortexed and centrifuged, the supernatant was analyzed by HPLC to determine whether the reaction was completed. Therein, the conditions for the HPLC analysis includes: analytical column: XBridge/C18/3.5 um/4.6*150 mm; elution conditions: B% (5%-75%), 0-30 min; column temperature: 50°C; and detection wavelengths: 210 nm and 260 nm.

The crude reaction product was purified. The purification process included: adding a 1 *PBS/acetonitrile solution (volume: 4 times the volume of the crude reaction product solution) to the crude reaction product solution at room temperature under shaking, and vortexing and centrifuging the mixture to remove the supernatant, to afford Conjugate 1 having the structure as shown in Formula (511) as a pellet.

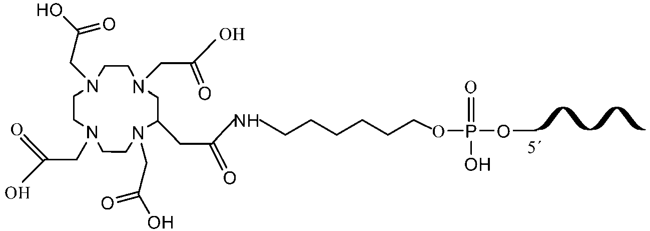

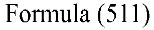

Conjugate 1 obtained in the above step was purified by using the Agilent semi-preparative reverse-phase purification method to afford 4 mg of purified Conjugate 1 (yield: 57.1%). In the Preparation Examples of the present disclosure, the calculation formula of the yield was: weight of the product / weight of the nucleotide sequence linked to the phosphoramidite monomer * 100%, and in this Preparation Example, the yield was 4 mg/7 mg * 100 = 57.1%.

The purification conditions includes: a column: XBridge Prep/C18/10 um, 19*250 mm; mobile phase PA: 100 mM TEAA (pH=7.1-7.3), acetonitrile; gradient B%: 5%-60%; 0-40 min.

4) The purified Conjugate 1 was dissolved in a 0.1 M acetate buffer solution (pH 4.5) to afford a solution of Conjugate 1 with a concentration of 1 mg/mL. To 1 nmol of the solution of Conjugate 1, 0.01 nmol of Lu177 (0.3 mCi) was added, and the mixture was placed in 10 µL of a 100 mM acetate buffer solution (pH 4.5) for gradient incubation. The gradient incubation process was as follows: incubation at 90°C for 30 min, followed by incubation at 60°C for 15 min, and finally incubation at 37°C for 20 min. The reaction mixture was purified by thin-layer chromatography (TLC), and the purity of the purified Conjugate RA1 was 92.9%. This Conjugate RA1 is the sodium salt of the compound having the structure as shown in Formula (521):

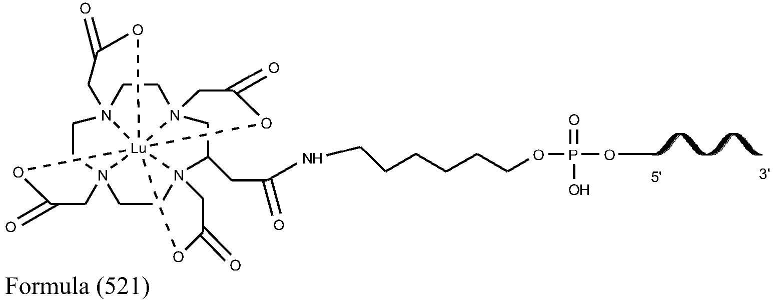

After completion of the synthesis of conjugate RA1, the resultant conjugate was diluted to a concentration of 0.2 mg/mL by using ultra-pure water (prepared by Milli-Q ultra-pure water instrument, resistivity 18.2 MΩ* cm (25°C)), and then molecular weight was determined by a LC-MS instrument (LC-MS, liquid chromatography-mass spectrometry, purchased from Waters Crop., model: LCT Premier). The results show that the calculated molecular weight is 8525.639 and the measured molecular weight is 8563.13; the measured value of the molecular weight is consistent with the calculated value, indicating that the target conjugate was obtained.

### Preparation Example 2 Synthesis of Conjugate RA2

In this Preparation Example, Conjugate RA2 was prepared according to the following preparation procedure:
(1) The nucleotide sequence as shown in SEQ ID NO: 24 was synthesized according to the procedure of step (1) in Preparation Example 1.
(2) According to the method of linking nucleoside phosphoramidite monomers, the phosphoramidite monomer containing HO-(CH₂)₆-S-S-(CH₂)₆- group (purchased from Hongene Biotech Company) was additionally linked to the 3' terminus of the nucleotide sequence linked to an amino terminus as shown in Formula (120), and the oligonucleotide single strand was cleaved from the solid phase support to afford the oligonucleotide single strand S1 as shown in Formula (220): 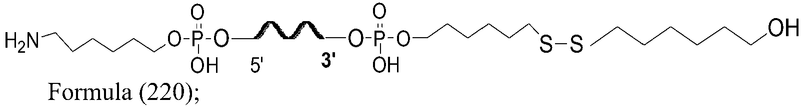 in the Formula, 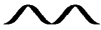 represents the oligonucleotide sequence corresponding to Conjugate RA1.

The compound as shown in Formula (220) obtained after ultrafiltered, desalting, and concentration (7 mg, 1.0 eq) was dissolved in 0.205 mL of an aqueous sodium bicarbonate solution (100 mM) to afford a nucleotide solution.

The compound as shown in Formula (230) (2.28 mg, 5.0 eq, CAS No. 127985-74-4, purchased from Shanghai Huayuan Century Trading Co., Ltd.) was dissolved in 0.82 mL of DMF; the resultant solution was mixed with the nucleotide solution obtained in the above step; and the mixed solution was added with 18 µL of triethylamine, mixed thoroughly by vortex, and then reacted in a constant temperature oscillating metal bath for 4 h to afford a crude reaction product.

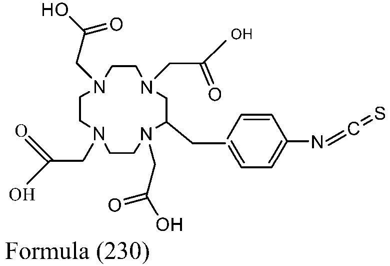

(3) The crude reaction product was purified according to the method of step (3) in Preparation Example 1 to afford 7 mg of Conjugate 2 having the structure as shown in Formula (512) (the yield can reach 100%, 7mg/7mg*100=100%):

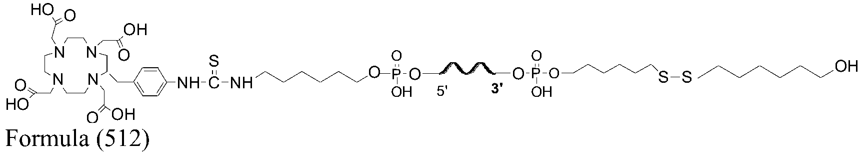

### (4) Conjugation of a Cy5 fluorophore

7 mg of Conjugate 2 was dissolved in 1 mL of purified water to afford a solution of Conjugate 2. 7 mg of tris(2-carboxyethyl)phosphine (TCEP) was dissolved in 1 mL of purified water to afford a tris(2-carboxyethyl)phosphine solution. All of the solution of Conjugate 2 and all of the tris(2-carboxyethyl)phosphine solution were mixed and reacted at room temperature for 2 h to afford 6.37 mg of the compound having the structure as shown in Formula (522) (yield 91%), designated as Conjugate 3.

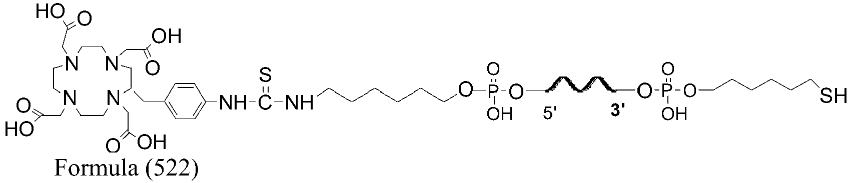

The compound as shown in Formula (522) obtained after ultrafiltered, desalting, and concentration (6.37 mg, 1.0 eq) was dissolved in 0.944 mL of an aqueous ammonium acetate solution (100 mM) to afford a solution of Conjugate 3.

The compound comprising a Cy5 fluorophore having the structure as shown in Formula (532) (2.30 mg, 5.0 eq) was dissolved in 0.944 mL of a DMF solution; the resultant solution was added to the solution of Conjugate 3; and the reaction was carried out at room temperature for 4 h to afford 3 mg of Conjugate 4 having the structure as shown in Formula (542) (yield 47.1%):

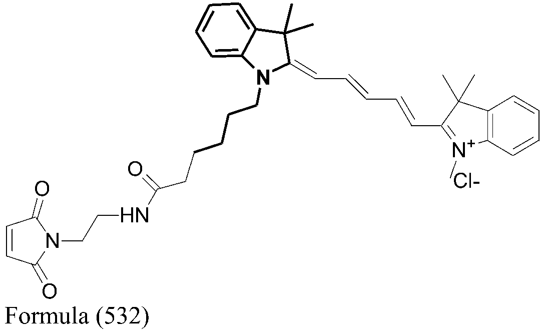

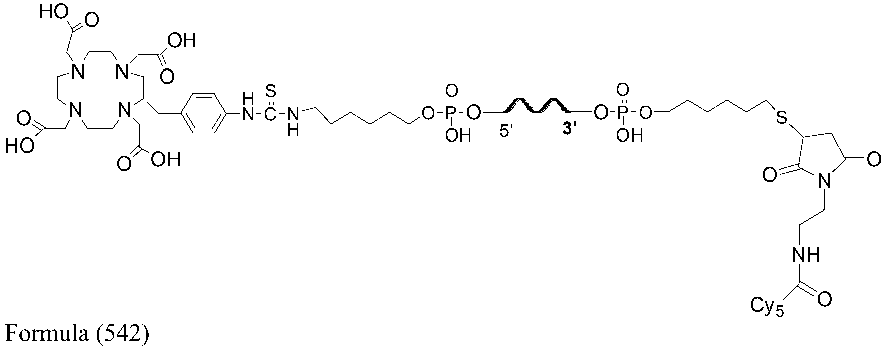

4) Conjugate 4 was dissolved in a 0.1 M acetate buffer solution (pH 5) to afford a solution of Conjugate 4 with a concentration of 1 mg/mL. To 1 nmol of the solution of Conjugate 4, 0.4 nmol of Lu177 was added, and the mixture reacted at 90°C for 15 min. The reaction mixture was purified by thin-layer chromatography to afford Conjugate RA2, which was the sodium salt of the compound having the structure as shown in Formula (552).

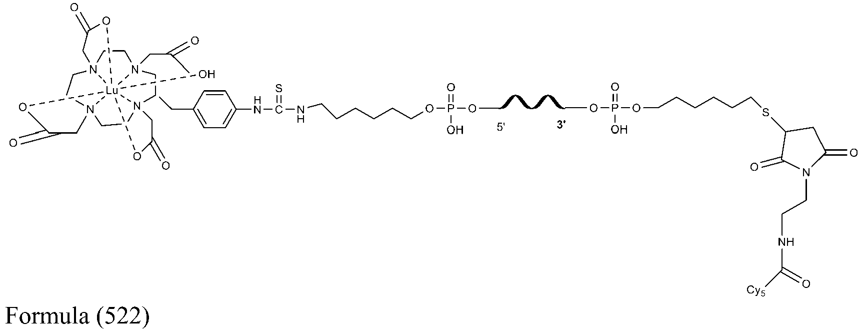

The molecular weight of Conjugate RA2 was determined according to the method in Preparation Example 1. The results show that the calculated molecular weight was 9528.404 and the measured molecular weight was 9527.23; the measured value of the molecular weight is consistent with the calculated value, indicating that the target conjugate was obtained.

### Preparation Example 3 Synthesis of Conjugate RA3

In this Preparation Example, Conjugate RA3 was prepared according to the following preparation procedure:
(1) The nucleotide sequence as shown in SEQ ID NO: 24 was synthesized according to the procedure of step (1) in Preparation Example 1.
(2) The nucleotide sequence linked to an amino terminus as shown in Formula (120) was prepared according to the method in Preparation Example 1, and dissolved according to the method in Preparation Example 1 to afford a nucleotide solution.

The compound as shown in Formula (220) obtained after ultrafiltered, desalting, and concentration (9.5 mg, 1.0 eq) was dissolved in 0.29 mL of an aqueous sodium bicarbonate solution (100 mM) to afford a nucleotide solution.

The compound as shown in Formula (230) (3.22 mg, 5.0 eq) was dissolved in 1.16 mL of DMF; the resultant solution was mixed with the nucleotide solution obtained in the above step; and the mixed solution was added with 25 µL of triethylamine, mixed thoroughly by vortex, and then reacted in a constant temperature oscillating metal bath for 4 h to afford a crude reaction product.

(3) The crude reaction product was purified according to the method of step (3) in Preparation Example 1 to afford 7.95 mg of Conjugate 5 having the structure as shown in Formula (513) (the yield was 83.7%, 7.95 mg/9.5 mg*100=83.7%):

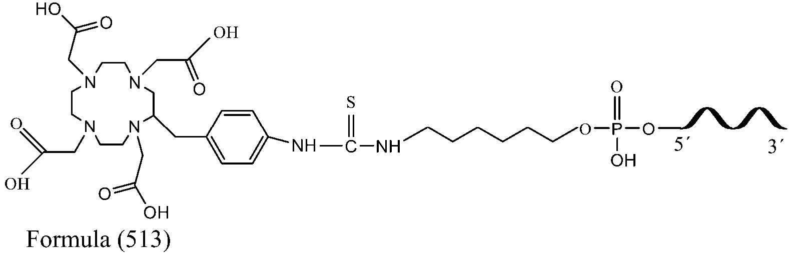

4) Conjugate 5 was dissolved in a 0.1 M acetate buffer solution (pH 5) to afford a solution of Conjugate 5 with a concentration of 1 mg/mL. To 1 nmol of the solution of Conjugate 5, 0.4 nmol of ¹⁷⁷Lu chloride (LuCl₃) was added, and the mixture reacted at 90°C for 15 min. The reaction mixture was purified by thin-layer chromatography to afford Conjugate RA3, which was the sodium salt of the compound having the structure as shown in Formula (523):

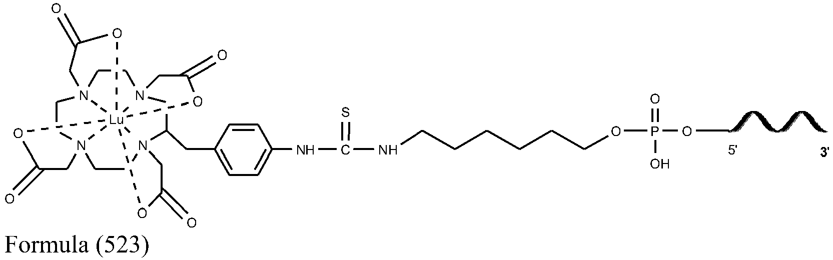

The molecular weight of Conjugate RA3 was determined according to the method in Preparation Example 1. The results show that the calculated molecular weight was 8690.949 and the measured molecular weight was 8689.65; the measured value of the molecular weight is consistent with the calculated value, indicating that the target conjugate was obtained.

### Preparation Example 4 Synthesis of Conjugate RA4

According to the method in Preparation Example 3, Conjugate 5 was dissolved in a 0.1 M acetate buffer solution (pH 5) to afford a solution of Conjugate 5 with a concentration of 1 mg/mL. 3 mL of the solution of Conjugate 5 and 17.3 µL of 10 mM ⁶⁴Cu chloride (⁶⁴CuCl₂) were mixed and reacted at 50°C for 10 min to afford Conjugate RA4. Conjugate RA4 was diluted with deionized water, and then subjected to LC-MS analysis/ The result show that the purity of Conjugate RA4 was 95.2%, and the calculated molecular weight is consistent with the measured molecular weight. Therein, Conjugate RA4 was the sodium salt of the compound having the structure as shown in Formula (514).

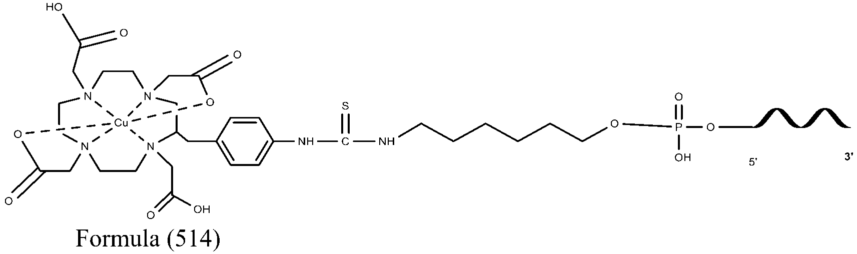

### Comparative Preparation Example 1: Synthesis of Comparative conjugate 1

This Comparative Preparation Example was performed according to the steps in Preparation Example 3, except that the nucleotide sequence as shown in SEQ ID NO: 24 in Preparation Example 3 was replaced with the nucleotide sequence as shown in SEQ ID NO: 44.

5'-GmGmAmCmAmUmGmGmAmUmUmCmUmUmGmUmCmUmGmUmGmUmC
mCm-3' (SEQ ID NO: 44)

The molecular weight of Comparative conjugate 1 was determined according to the method in Preparation Example 1. The results show that the calculated molecular weight was 8690.949 and the measured molecular weight was 8689.79; the measured value of the molecular weight is consistent with the calculated value, indicating that the target conjugate was obtained.

### Comparative Preparation Example 2: Synthesis of Chelate 1

1 nmol of DOTA chelating agent was mixed with 0.01 nmol of Lu177 (0.3 mCi), and the mixture was incubated in 10 µL of an acetate buffer solution (pH 4.5, 100 mM) at 95°C for 30 min to afford Chelate 1, a conjugate comprising the radionuclide Lu177.

### Experimental Example 1: In vitro targeting ability of conjugate RA1 against subcutaneous tumors

Two mice were prepared: one inoculated with subcutaneous U118 tumors of 1000 mm³ and the other inoculated with subcutaneous U118 tumors of 300 mm³.

Conjugate RA1 was diluted with a PBS buffer solution, and respectively administered to the two mice by tail-vein injection at a dose of 0.5 mg/kg and 15 mCi Lu¹⁷⁷/kg, based on the amount of the nucleotide sequence in the conjugate.

Blood samples were collected via orbital bleeding at 2 h and 4 h post-administration, and the radiation levels (mCi/mL) in the blood were measured after administration.

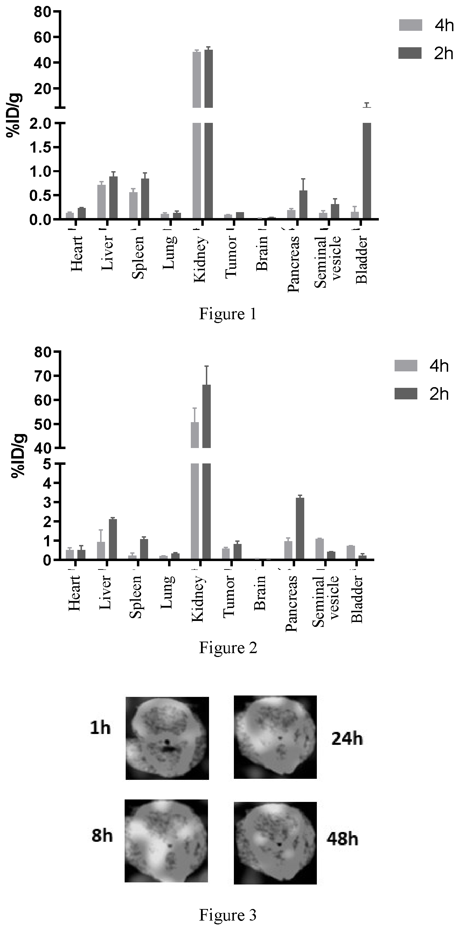

Figure 1 shows a bar chart showing the *ex-vivo* quantitative analysis at 2h and 4h after injection of Conjugate RA1 into the model mice inoculated with subcutaneous U118 tumors of 300 mm³. Figure 2 shows a bar chart showing the *ex-vivo* quantitative analysis at 2h and 4h after injection of Conjugate RA1 into the model mice inoculated with subcutaneous U118 tumors of 1,000 mm³. As can be seen from Figures 1 and 2, the conjugates of the present disclosure exhibit a certain degree of tumor-targeting effect in tumor tissues.

The results in Figures 1 and 2 show that Conjugate RA1 was enriched in the subcutaneous U118 tumors of 300 mm³ and the subcutaneous U118 tumors of 1000 mm³, and show greater enrichment in the subcutaneous U118 tumors with a larger area of 1000 mm³.

### Experimental Example 2: Targeting ability of Conjugate RA1 toward in situ tumors

Two mice were prepared, and both were inoculated with *in situ* U118 tumors. Conjugate RA1 was diluted with a PBS buffer solution, and respectively administered to the two mice by tail-vein injection at a dose of 0.5 mg/kg and 15 mCi Lu¹⁷⁷/kg, based on the amount of the nucleotide sequence in the conjugate.

SPECT-CT scans were respectively performed on the mice at 1 h, 8 h, 24 h, and 48 h post-administration. Figure 3 shows the front-view SPECT-CT scan images of mice at 1 h, 8 h, 24 h, and 48 h post-administration. As can be seen from Figure 3, significant radiation signals were detected at the *in situ* U118 tumor sites over a period of 48 hours after administration of Conjugate RA1 of the present disclosure, indicating that Conjugate RA1 comprising the nucleotide sequence of the present disclosure can target *in situ* U118 tumors.

### Experimental Example 3: Targeting ability of Conjugate RA3 toward subcutaneous tumors

Two mice were prepared: one was the test group mouse and the other was the control group mouse, and both were inoculated with subcutaneous U118 tumors of 300 mm³.

Conjugate RA3 was diluted with a PBS buffer solution and administered to the test group mouse by tail-vein injection at a dose of 8 µg/ nmol and 2 mCi Lu¹⁷⁷/nmol, based on the amount of the nucleotide sequence in the conjugate; free Lu177 was administered to the control group mouse by tail-vein injection at a dose of 0.8 mCi Lu¹⁷⁷/nmol.

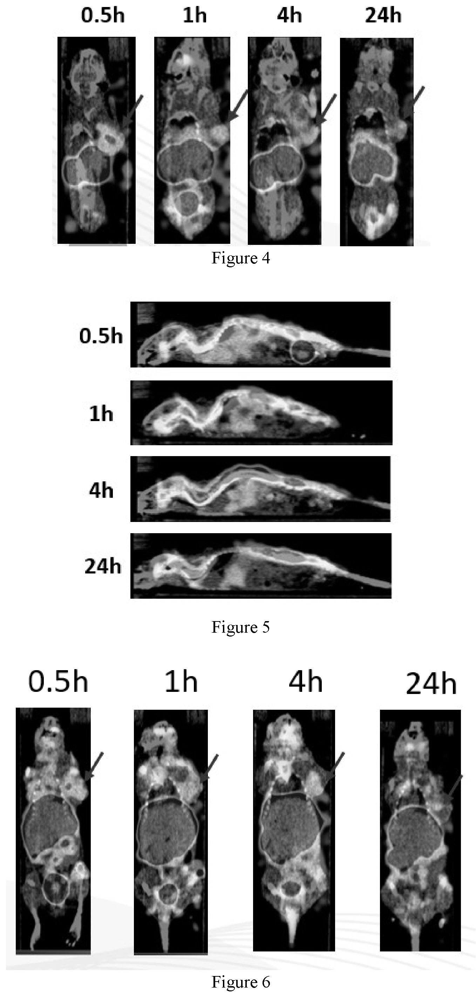

SPECT-CT scans were respectively performed on the two groups of mice at 0.5 h, 1 h, 4 h, and 24 h post-administration. Figure 4 shows the top-view SPECT-CT scan images of the test group mouse at 0.5 h, 1 h, 4 h, and 24 post-administration. Figure 5 shows the side-view SPECT-CT scan images of the control group mouse at 0.5 h, 1 h, 4 h, and 24 post-administration. As can be seen from Figure 4, significant radiation signals were detected in the subcutaneous U118 tumors over a period of 24 hours after administration of Conjugate RA3 of the present disclosure, and except that the metabolic organ kidney exhibited radiation signals, there was no significant enrichment in other organs. In contrast, Figure 5 shows that in the mouse injected with free Lu177, radiation signals with no significant difference were detected across all organs and tissues, showing no tumor-targeting ability. This demonstrates that Conjugate RA3 comprising the nucleotide sequence of the present disclosure can specifically target subcutaneous U118 tumors.

### Experimental Example 4: Targeting ability of Conjugate RA2 towards subcutaneous tumors

One mouse was prepared and inoculated with subcutaneous U118 tumors of 300 mm³. Conjugate RA2 was diluted with a PBS buffer solution and administered to the mouse by tail-vein injection at a dose of 2 µg/ nmol and 500 µCi Lu¹⁷⁷/nmol, based on the amount of the nucleotide sequence in the conjugate.

SPECT-CT scans were respectively performed on the mouse at 0.5 h, 1 h, 4 h, and 24 h post-administration. Figure 6 shows the top-view SPECT-CT scan images of the mouse at 0.5 h, 1 h, 4 h, and 24 post-administration, wherein the site indicated by the red arrow is the site of the subcutaneous U118 tumors. As can be seen from Figure 6, radiation signals were detected in the subcutaneous U118 tumors over a period of 24 hours after administration of Conjugate RA2 of the present disclosure, This demonstrates that Conjugate RA2 comprising the nucleotide sequence of the present disclosure can exert effects in the subcutaneous U118 tumors over a longer period of time.

Some embodiments of the present disclosure are described in detail above, but the present disclosure is not limited to the specific details of the above embodiments. Various simple variations of the technical solutions of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, various possible combinations are no longer described in the present disclosure.

In addition, various different embodiments of the present disclosure can also be arbitrarily combined as long as it does not deviate from the idea of the present disclosure, which should also be regarded as the disclosure of the present disclosure.

## Claims

1. A conjugate comprising one or more delivery groups and one or more chelating agent-derived groups; wherein each of the chelating agent-derived groups is independently formed by removing one or more atoms or one or more functional groups from a chelating agent, wherein the chelating agent is a chelating agent capable of forming a chelate with a radionuclide;
each of the delivery groups is independently formed by removing one or more hydrogen atoms or one or more functional groups from an aptamer, wherein the aptamer comprises a segment of a contiguous nucleotide sequence, wherein the group linking two adjacent nucleotides is independently a phosphate ester group or a phosphate ester group with modification group(s), each nucleotide is one selected from modified nucleotides or unmodified nucleotides, and the contiguous nucleotide sequence has the sequence as shown in Formula (1):
5'- T₁-S₁-Nₐ-S₂-N_{b}-S₃-N_{c}-S₄-T₂-3' Formula (1)
wherein T₁ is a motif consisting of 1-3 nucleotides, T₂ is a motif consisting of 0-15 nucleotides, and T₂ does not comprise a motif that is completely reverse complementary to T₁;
S₁ and S₄ are each motifs consisting of 3-7 nucleotides, S₁ and S₄ are of the same length and are completely reverse complementary to each other;
Nₐ and N_{c} are each motifs consisting of 1-4 nucleotides, each nucleotide in Nₐ is not complementary to each nucleotide in N_{c}, and the total number of U in Nₐ and N_{c} accounts for more than 50% of the total number of all nucleotides in Nₐ and N_{c};
S₂ and S₃ are each motifs consisting of 1-4 nucleotides, S₂ and S₃ are of the same length and are completely reverse complementary to each other;
N_{b} is a motif consisting of 3-6 nucleotides, and the nucleotides at both ends of N_{b} do not form AU or GC complementarity;
each of the delivery groups is independently linked to at least one of the chelating agent-derived groups or other delivery groups via at least one linking group, and each of the chelating agent-derived groups is independently linked to at least one of the delivery groups or at least one additional chelating agent-derived group via at least one linking group.

2. The conjugate according to claim 1, wherein the length of the contiguous nucleotide sequence is 18-50 nucleotides, or 20-40 nucleotides, or 21-36 nucleotides, or 24-32 nucleotides.

3. The conjugate according to claim 1 or 2, wherein T₁ consists of 2 nucleotides; or T₁ consists of 2 nucleotides and comprises at least one C; or
in a 5'-3' direction, T₁ is CU, UC or AC.

4. The conjugate according to claim 1 or 2, wherein T₂ consists of 0-10 nucleotides; or in a 5'-3' direction, T₂ consists of 1-9 nucleotides starting with U.

5. The conjugate according to claim 1, wherein S₁ and S₄ each consist of 3-5 nucleotides and are of the same length; or
in the reverse complementarity formed between S₁ and S₄, GC complementarity accounts for at least 40% of the total number of complementarity; or
in a 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

6. The conjugate according to claim 1, wherein the sum of the number of nucleotides in Nₐ and N_{c} is an integer of 2 to 4; or
the sum of the number of nucleotides in Nₐ and N_{c} is 3 or 4, and the sum of the number of U in Nₐ and N_{c} is 2 or 3; or
in a 5'-3' direction, Nₐ or N_{c} is independently U, UU, UC, or CU.

7. The conjugate according to claim 1, wherein S₂ and S₃ each consist of 2-3 nucleotides and are of the same length; or
the reverse complementarity formed between S₂ and S₃ comprises at least one GC complementarity; or
in a 5'-3' direction, S₂ is CA and S₃ is UG, or S₂ is AC and S₃ is GU, or S₂ is GCC and S₃ is GGU.

8. The conjugate according to claim 1, wherein N_{b} consists of 4 or 5 nucleotides; or in a 5'-3' direction, N_{b} is GACG, GACGU, GACCG, UACU, GUUG, or GAUCU.

9. The conjugate according to claim 1, wherein the contiguous nucleotide sequence has the sequence as shown in SEQ ID NO: 1, SEQ ID NO: 2 or SEQ ID NO: 3.

10. The conjugate according to claim 1, wherein the contiguous nucleotide sequence has the nucleotide sequence as shown in SEQ ID NO: 4:
5'-N₆GGAGUUCAN₁N₂N₃N₄UGN₅GCUCN₇-3' (SEQ ID NO: 4)
wherein N₁, N₂, and N₃ independently of one another are one of A, U, C, and G; N₄ is U, C or G or a motif consisting of two of U, C and G; N₅ is U, CU or UU; N₆ is CU, UC or AC; N₇ is U, UU or UUN₈; N₈ is a motif consisting of 1-15 nucleotides.

11. The conjugate according to claim 10, wherein the motif N₁N₂N₃N₄ consisting of N₁, N₂, N₃, and N₄ is one of GACG, GACGU, GACCG, UACU, GUUG, and GAUCU.

12. The conjugate according to claim 10 or 11, wherein the contiguous nucleotide sequence has the nucleotide sequence as shown in any one of SEQ ID NOs: 5-11.

13. The conjugate according to claim 10, wherein N₈ is a motif consisting of 1-8 nucleotides; or
in a 5'-3' direction, the nucleotide sequence of N₈ is CCGAUCUC; or
the contiguous nucleotide sequence has the sequence as shown in any one of SEQ ID NOs: 12-14.

14. The conjugate according to claim 1, wherein each cytidine nucleotide in the contiguous nucleotide sequence is a fluoro modified cytidine nucleotide, and/or each uridine nucleotide in the contiguous nucleotide sequence is a fluoro modified uridine nucleotide; or
each nucleotide in the contiguous nucleotide sequence is a 2'-methoxy modified nucleotide; or
one or more uridine nucleotides in the motifs N_{b} and S₃ in the contiguous nucleotide sequence have a modified base.

15. The conjugate according to claim 14, wherein the contiguous nucleotide sequence has the nucleotide sequence as shown in one of SEQ ID NOs: 15-33.

16. The conjugate according to claim 1, wherein at least one group linking two adjacent nucleotides in the contiguous nucleotide sequence is a phosphorothioate group, or each group linking two adjacent nucleotides is a phosphorothioate group.

17. The conjugate according to claim 16, wherein the contiguous nucleotide sequence has the nucleotide sequence as shown in one of SEQ ID NOs: 34-39.

18. The conjugate according to claim 17, wherein the conjugate has the structure as shown in Formula (101): 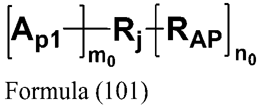
wherein each R_{AP} group is independently a group having the structure as shown in Formula (102): 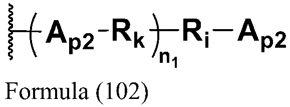
wherein each A_{P1} group and each A_{P2} group is identical or different, and independently and respectively represents one of the delivery groups or one of the chelating agent-derived groups, and the conjugate comprises at least one of the delivery groups and at least one of the chelating agent-derived groups; Rⱼ represents a linking group; each Rₖ or each Rᵢ is identical or different, and independently and respectively represents a covalent bond or a linking group, and Rᵢ and Rₖ are not covalent bonds at the same time; each n₁ independently of one another represents an integer of 0 to 4;
m₀ is an integer of 1 to 6; n₀ is an integer of 1 to 6; and 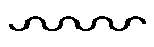 represents the site where the group is covalently linked.

19. The conjugate according to claim 18, wherein m₀ is an integer of 1 to 4, and/or n₀ is an integer of 1 to 3, and/or each n₁ is independently an integer of 0 to 1; or
m₀ is 1, A_{P1} is the chelating agent-derived group, n₀ is 1, n₁ is 0, and A_{P2} is the delivery group.

20. The conjugate according to claim 18 or 19, wherein each of the Rₖ or each of the Rᵢ is independently a covalent bond or a straight-chain alkylene group having a length of 1 to 70 carbon atoms, or one or more carbon atoms in the straight-chain alkylene group are replaced by one or more groups selected from the group consisting of: C(O), NH, O, S, C(S), CH=N, S(O)₂, OP(O)₂, OP(O)(S), C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene group can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl).

21. The conjugate according to claim 20, wherein each n₁ is 0, and each Rᵢ is independently a covalent bond, or any one of the following linking groups, or a linking combination of two or more of the following linking groups: C₁-C₂₀ alkylene, phosphate ester bond, phosphorothioate bond, amide bond, ester bond, ether bond, thioether bond, disulfide bond, 1,2,3-triazolylene group, a polyethylene glycol residue, pyrrolidinylene group, 2-oxopyrrolidinylene group, phenylene, cyclohexylene, 2-succinimidylene group, 2-thiosuccinimidylene group, an amino acid residue, and a nucleotide residue.

22. The conjugate according to claim 21, wherein each Rᵢ is independently any one of the following groups, or a linking combination of two of the following groups: a covalent bond, a disulfide bond, propylene phosphate ester group, 2-thiosuccinimidylene group, an amino acid residue, or a GAU trinucleotide residue.

23. The conjugate according to any one of claims 18-22, wherein Rⱼ is a covalent bond and m₀ is 1.

24. The conjugate according to any one of claims 18-22, wherein Rⱼ is a linking group, wherein the linking group is a straight-chain alkylene group having a length of 1 to 70 carbon atoms, or one or more carbon atoms in the straight-chain alkylene group are replaced by one or more groups selected from the group consisting of: C(O), NH, O, S, C(S), CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene group can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl).

25. The conjugate according to any one of claims 18-22, wherein Rⱼ is a linking group, wherein the linking group Rⱼ comprises a backbone moiety, a side chain moiety and a conjugation linking moiety, wherein the backbone moiety is respectively linked to the conjugation linking moiety and the side chain moiety, each of the side chain moieties is respectively linked to the backbone moiety and the R_{AP} group, each of the conjugation linking moieties is respectively linked to the backbone moiety and the chelating agent-derived group A₀, wherein
the backbone moiety is a straight-chain alkylene group having a length of 1 to 70 carbon atoms, or one or more carbon atoms in the straight-chain alkylene group are replaced by one or more groups selected from the group consisting of: C(O), C(S), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and wherein the straight-chain alkylene group can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, - NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl);
each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and the straight-chain alkylene group can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl);
each of the conjugation linking moieties is independently a covalent bond, or any one of the following linking structures, or a linking combination of two or more of the following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol residue, pyrrolidinylene group, 2-oxopyrrolidinylene group, phenylene, cyclohexylene, 2-succinimidylene group, 2-thiosuccinimidylene group, an amino acid residue, and a nucleotide residue.

26. The conjugate according to claim 25, wherein each of the conjugation linking moieties in the linking group Rⱼ is respectively linked to the backbone moiety and one of the chelating agent-derived groups A₀; the number of side chain moieties is n₀, and each of the side chain moieties is respectively linked to the backbone moiety and one of the R_{AP} groups.

27. The conjugate according to claim 25 or 26, wherein all of the side chain moieties are linked to the same atom in the backbone moiety; or, each of the side chain moieties is linked to a different atom in the backbone moiety.

28. The conjugate according to claim 27, wherein m₀ is 1, and the linking group Rⱼ comprises the structure as shown in Formula (301): 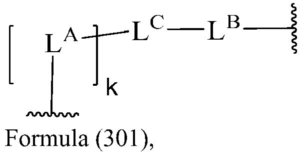
wherein k is an integer of 1 to 3; L^{C} is the backbone moiety, L^{A} is the side chain moiety, L^{B} is the conjugation linking moiety, and 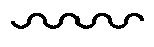 represents the site where the group is covalently linked;
the backbone moiety L^{C} is a covalent bond or a 2- to 4-valence, straight-chain or branched C₁-C₂₅ saturated hydrocarbyl, or one or more carbon atoms in the saturated hydrocarbyl are replaced by one or more groups selected from the group consisting of: C(O), C(S), NH, O, S, CH=N, S(O)₂, OP(O)₂, C₅-C₈ glycosidylene group, C₂-C₅ alkenylene group, C₂-C₅ alkynylene group, C₆-C₁₀ arylene group, C₃-C₈ heterocyclylene group, and C₅-C₁₀ heteroarylene group; wherein the saturated hydrocarbyl can have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, - C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), - N(C₁-C₅ alkyl)C(O)(C₁-C₅ alkyl), -N(C₁-C₅ alkyl)C(O)(phenyl), -C(O)C₁-C₅ alkyl, - C(O)C₁-C₅ alkylphenyl, -OC(O)C₁-C₅ alkyl, -SO₂(C₁-C₅ alkyl), -SO₂(phenyl), - SO₂NH₂, -SO₂NH(C₁-C₅ alkyl), -SO₂NH(phenyl), -NHSO₂(C₁-C₅ alkyl), and - NHSO₂(phenyl);
each of the side chain moieties is independently a covalent bond, or a straight-chain alkylene group having a length of 1 to 70 carbon atoms, or one or more carbon atoms in the straight-chain alkylene group are replaced by one or more groups selected from the group consisting of: C(O), NH, O, S, CH=N, S(O)₂, OP(O)₂, C₅-C₈ glycosidylene group, C₂-C₁₀ alkenylene group, C₂-C₁₀ alkynylene group, C₆-C₁₀ arylene group, C₃-C₁₈ heterocyclylene group, and C₅-C₁₀ heteroarylene group; and the straight-chain alkylene group can have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, - SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), - N(C₁-C₁₀ alkyl)(C₁-C₁₀ alkylphenyl), -NH(C₁-C₁₀ alkylphenyl), cyano, nitro, -CO₂H, - C(O)O(C₁-C₁₀ alkyl), -CON(C₁-C₁₀ alkyl)(C₁-C₁₀ alkyl), -CONH(C₁-C₁₀ alkyl), - CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, - C(O)C₁-C₁₀ haloalkyl, -OC(O)C₁-C₁₀ alkyl, -SO₂(C₁-C₁₀ alkyl), -SO₂(phenyl), - SO₂(C₁-C₁₀ haloalkyl), -SO₂NH₂, -SO₂NH(C₁-C₁₀ alkyl), -SO₂NH(phenyl), - NHSO₂(C₁-C₁₀ alkyl), -NHSO₂(phenyl), and -NHSO₂(C₁-C₁₀ haloalkyl);
each of the conjugation linking moieties is independently a covalent bond, or any one of the following structures, or a linking combination of two or more of the following linking structures: C₁-C₁₀ straight-chain alkylene, phosphate ester bond, phosphorothioate bond, amide bond, ester bond, ether bond, disulfide bond, 1,2,3-triazolylene group, a polyethylene glycol residue, pyrrolidinylene group, 2-oxopyrrolidinylene group, phenylene, cyclohexylene, 2-succinimidylene group, 2-thiosuccinimidylene group, an amino acid residue, and a nucleotide residue.

29. The conjugate according to claim 28, wherein the conjugate has the structure as shown in Formula (305): 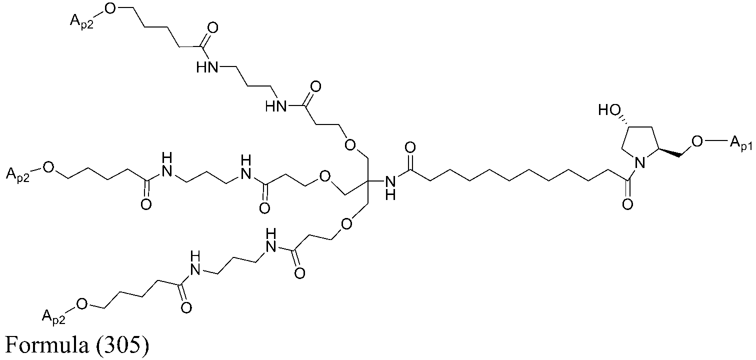 wherein A_{P1} is the chelating agent-derived group, and A_{P2} is the delivery group.

30. The conjugate according to claim 28, wherein the linking group Rⱼ has the structure as shown in Formula (306): 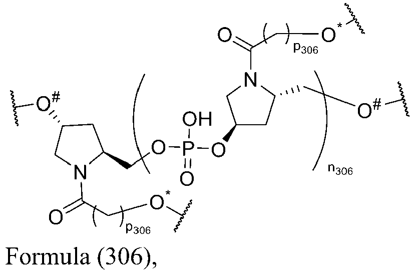
wherein n₃₀₆ is an integer of 0-3, each p₃₀₆ is independently an integer of 1 to 6, 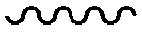 represents the site where the group is covalently linked; the oxygen atom marked by * and the R_{AP} group form a phosphate ester bond, an ether bond or an ester bond linkage; at least one of the oxygen atoms marked by # is linked to the chelating agent-derived group by forming an ether bond, an ester bond or a phosphate ester bond;
and the remaining oxygen atom marked by # is linked to a hydrogen atom to form a hydroxyl group, or is linked to a C₁-C₃ alkyl group to form a C₁-C₃ alkoxy group.

31. The conjugate according to claim 30, wherein the conjugate has the structure as shown in Formula (307a), (307b) or (307c): 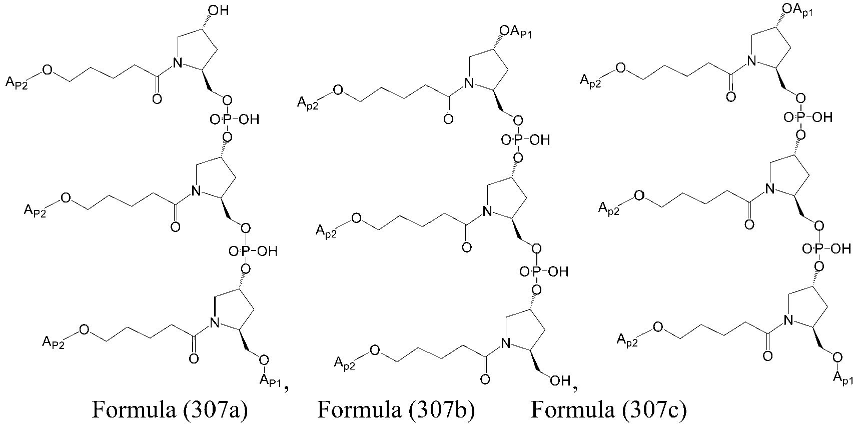 wherein A_{P1} is the chelating agent-derived group, and A_{P2} is the delivery group.

32. The conjugate according to claim 28, wherein the conjugate has the structure as shown in Formula (308): 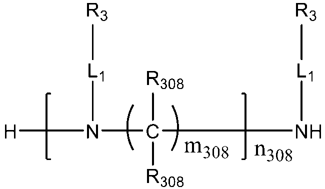 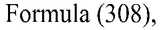
wherein n₃₀₈ is an integer selected from 1 to 10;
each m₃₀₈ is independently an integer selected from 2 to 10;
each R₃₀₈ is independently H, C₁-C₁₀ alkyl, C₁-C₁₀ haloalkyl, or C₁-C₁₀ alkoxy;
each R₃ is independently the A_{P1} group, or is the R_{AP} group, and at least one R₃ is the A_{P1} group, and at least one R₃ is the R_{AP} group; or one R₃ is the A_{P1} group, and the remaining R₃ groups are the R_{AP} groups;
each L₁ linked to the A_{P1} group represents the conjugation linking moiety, and each L₁ linked to the R_{AP} represents the side chain moiety.

33. The conjugate according to claim 32, wherein each L₁ is independently selected from the group consisting of the groups L4-L23 and any linking combination thereof.

34. The conjugate according to claim 33, wherein each L₁ is independently selected from the group consisting of linking combinations of at least two of the groups L4-L9, L13, L14, and L18; or
each L₁ is independently a linking combination of at least two of the groups L4, L5, L7, L9, L13, L14, and L18.

35. The conjugate according to any one of claims 32-34, wherein the length of each
L₁ is independently 3-25 atoms; or
the length of each L₁ is independently 4-15 atoms.

36. The conjugate according to any one of claims 32-35, wherein n₃₀₈ is an integer of 2 to 6, two to four of the R₃ groups are the R_{AP} groups, and the remaining R₃ groups are the chelating agent-derived groups.

37. The conjugate according to any one of claims 32-36, wherein each m₃₀₈ independently of one another is an integer of 2 to 5, and/or each m₃₀₈ is equal.

38. The conjugate according to any one of claims 32-37, wherein n₃₀₈ is an integer selected 2 to 4; each m₃₀₈ is independently an integer selected from 2 to 4; and each R₃₀₈ is H.

39. The conjugate according to claim 38, wherein one R₃ is the chelating agent-derived group and the remaining R₃ groups are the R_{AP} groups.

40. The conjugate according to claim 38 or 39, wherein each L₁ comprises both an attachment site linked to the N atom of the nitrogen-containing backbone and an attachment site linked to the A_{P1} group or the R_{AP} group, and the site linked to the N atom of the nitrogen-containing backbone and the N atom form an amide bond; or at least one L₁ comprises a first function group and an optional second functional group, wherein the first functional group can react with a group on an oligonucleotide or nucleotide to form a phosphate ester bond or phosphorothioate bond linkage, and the second functional group is a functional group formed after cleavage of the covalent linkage with the solid phase support; or
one or more L₁ groups are selected from B5, B6, B5', or B6': 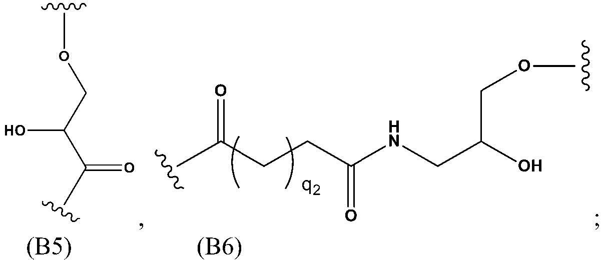 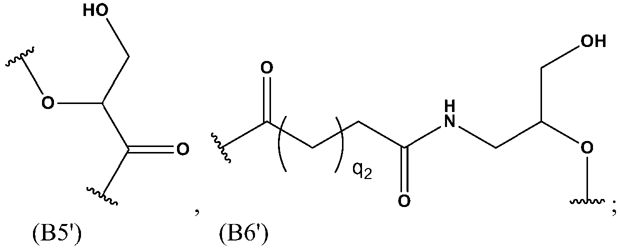
wherein 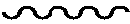 represents the site where the group is covalently linked, q₂ is an integer of 1 to 10; or q₂ is an integer of 1 to 5.

41. The conjugate according to any one of claims 32-40, wherein the conjugate has the structure as shown in Formula (403), (404), (405), (406), (407), (408), (409), (410), (411), (412), (413), (414), (415), (416), (417), (418), (419), (420), (421), (422), (423), (424), (425), (426), or (427).

42. The conjugate according to any one of claims 18-22, wherein Rj comprises a nucleotide sequence I and a nucleotide sequence II, wherein the nucleotide sequence I and the nucleotide sequence II each comprise 5 to 25 modified or unmodified nucleotides, and the nucleotide sequence I and the nucleotide sequence II are at least partially reverse complementary to each other; the delivery group is linked to the nucleotide sequence I, the chelating agent-derived group is linked to the nucleotide sequence II, and the nucleotide sequence I and the nucleotide sequence II do not elicit an immune response or a toxic reaction in a subject.

43. The conjugate according to claim 42, wherein the 3' terminus of the delivery group is linked to the 5' position of the ribose of the 5' terminal nucleotide of the nucleotide sequence I via a phosphate ester bond, and the chelating agent-derived group is linked to the 5' position of the ribose of the 5' terminal nucleotide of the nucleotide sequence II; or
the chelating agent-derived group comprises a segment of a nucleotide sequence, and the 3' terminus of the nucleotide sequence is linked to the 5' position of the ribose of the 5' terminal nucleotide of the nucleotide sequence II via a phosphate ester bond.

44. The conjugate according to claim 43, wherein the nucleotide sequence I and the nucleotide sequence II are substantially reverse complementary or completely reverse complementary to each other; or
the lengths of the nucleotide sequence I and the nucleotide sequence II are equal and are both 10-20 modified or unmodified nucleotides; or
the nucleotide sequence I and the nucleotide sequence II both consist of 17 nucleotides and are completely reverse complementary to each other; or the nucleotide sequence I and the nucleotide sequence II respectively have the sequences as shown in SEQ ID NO: 40 and SEQ ID NO: 41:
5'- GUACAUUCUAGAUAGCC -3' (SEQ ID NO: 40)
5'- GGCUAUCUAGAAUGUAC -3' (SEQ ID NO: 41), or
the nucleotide sequence I and the nucleotide sequence II respectively have the sequences as shown in SEQ ID NO: 42 and SEQ ID NO: 43:
5'- GmUfAmCfAmUfUfCfUfAmGmAmUfAmGmCfCf-3' (SEQ ID NO: 42)
5'- GmGmCfUfAmUfCfUfAmGmAmAmUfGmUfAmCf-3' (SEQ ID NO: 43).

45. The conjugate according to any one of claims 18-44, wherein Rⱼ is cleavable.

46. The conjugate according to any one of claims 1-45, wherein each of the chelating agent-derived groups is independently selected from the group consisting of the groups formed by removing one atom or functional group from one of DOTA, HBED-cc, NOTA, TRAP, TETA, and DiAmSar;
optionally, each of the chelating agent-derived groups is independently a group formed by removing one atom or functional group from DOTA or NOTA.

47. The conjugate according to claim 18, wherein the conjugate has the structure as shown in Formula (501), (502), (503), or (504): 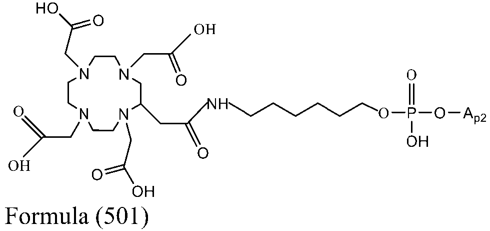 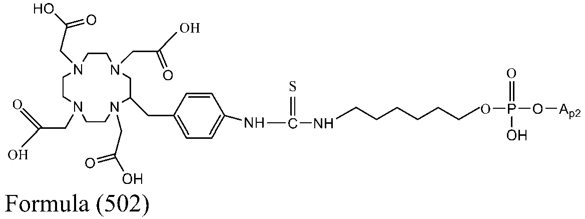 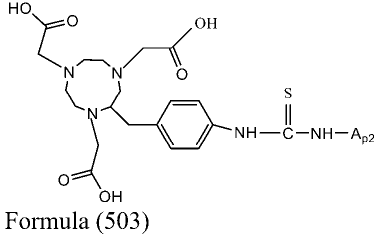 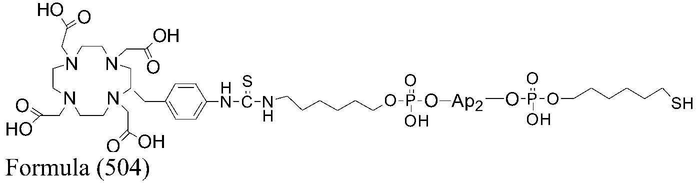 wherein the A_{P2} group is the delivery group.

48. The conjugate according to any one of claims 1-47, wherein the conjugate further comprises a diagnostic agent group, and the diagnostic agent group is linked to the delivery group via a covalent bond or a linking group; or
the diagnostic agent group is linked to the chelating agent-derived group via a covalent bond or a linking group.

49. The conjugate according to claim 48, wherein each of the diagnostic agent groups is independently selected from a contrast agent group or a fluorescence labelling tracer group; or
the diagnostic agent group is a Cy5 fluorophore or a Cy3 fluorophore.

50. A pharmaceutical conjugate comprising the conjugate according to any one of claims 1-49 and a radionuclide, wherein the radionuclide is a radioactive metal cation, and the radionuclide is linked to the chelating agent-derived group via a coordination bond.

51. The pharmaceutical conjugate according to claim 50, wherein the radionuclide is selected from the cations formed by at least one of ⁶⁸Ga, ¹⁷⁷Lu, ⁹⁰Y, ⁸⁹Zr, ^{99m}Tc, and ⁶⁴Cu;
optionally, the radionuclide is a cation formed by ⁶⁴Cu, ⁶⁸Ga or ¹⁷⁷Lu.

52. The pharmaceutical conjugate according to claim 51, wherein the pharmaceutical conjugate has the structure as shown in Formula (611), (612), (613), (614), or (615):

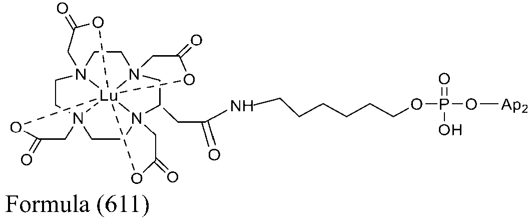

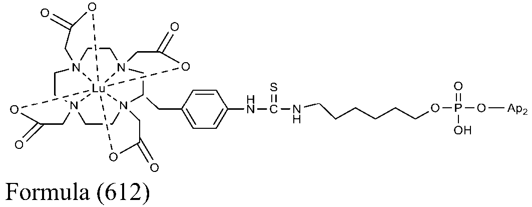

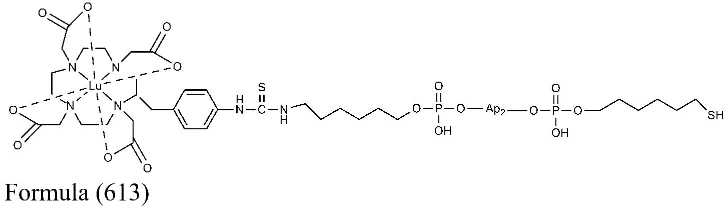

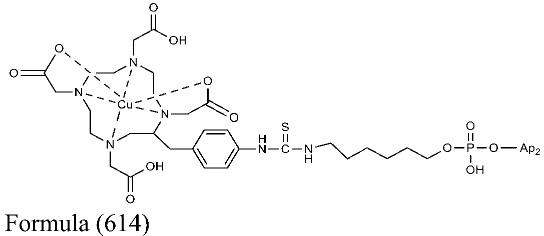

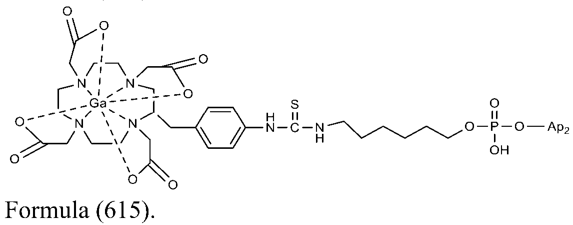

53. A pharmaceutically acceptable salt of the conjugate according to any one of claims 1-52;
alternatively, the pharmaceutically acceptable salt is a partial or completely water-soluble salt of the conjugate; or
the water-soluble salt is an amine salt or an alkali metal salt; or
the amine salt is one or more selected from the group consisting of an ammonium salt, a methylamine salt, a tertiary amine salt, and a quaternary ammonium salt, and the alkali metal salt is selected from a potassium salt or a sodium salt; or
the tertiary amine salt is a triethylamine salt, a triisopropylamine salt or an N,N-diisopropylethylamine salt; or
the pharmaceutically acceptable salt is a sodium salt or a partial sodium salt of the conjugate.

54. A pharmaceutical composition comprising the pharmaceutical conjugate according to any one of claims 50-52 or the pharmaceutically acceptable salt according to claim 53, and a pharmaceutically acceptable carrier.

55. The pharmaceutical composition according to claim 54, wherein the carrier is one or more selected from the group consisting of a solvent, a protectant, an osmotic pressure regulator, and other pharmaceutically acceptable excipients; and the weight ratio of the pharmaceutical conjugate to the carrier is 1: (1-500), and preferably 1: (1-50).

56. Use of one or more of the pharmaceutical conjugate according to any one of claims 50-52, the pharmaceutically acceptable salt according to claim 53, and the pharmaceutical composition according to claim 53 or 54 in the manufacture of a medicament for diagnosing and/or treating tumors and tumor-related diseases or symptoms.

57. The use according to claim 56, wherein the tumor is one or more of glioma, renal carcinoma and lung carcinoma.

58. A method for diagnosing and/or treating tumors and tumor-related diseases or symptoms, comprising administering an effective amount of the pharmaceutical conjugate according to any one of claims 50-52, the pharmaceutically acceptable salt according to claim 53, and/or the pharmaceutical composition according to claim 54 or 55 to a subject in need thereof.

59. The method according to claim 58, wherein the tumor is one or more of glioma, renal carcinoma and lung carcinoma.

60. A kit comprising one or more of the pharmaceutical conjugate according to any one of claims 50-52, the pharmaceutically acceptable salt according to claim 53, and the pharmaceutical composition according to claim 54 or 55.
